# EUROPEAN PATENT SPECIFICATION

(11) **EP 4 400 127 B1**
(45) Date of publication and mention of the grant of the patent: **25.11.2026**
(21) Application number: 24178842.1
(22) Date of filing: 28.01.2021
(51) Int. Cl.: A61M 60/122

(54) **INTRAVASCULAR BLOOD PUMP**
INTRAVASKULÄRE BLUTPUMPE
POMPE SANGUINE INTRAVASCULAIRE

(30) Priority: 31.01.2020 EP 20154829
(43) Date of publication of application: 17.07.2024
(62) Document number(s) of the earlier application(s) in accordance with Art. 76 EPC: 21702467.8 / 4 096 733
(73) Proprietor: ECP Entwicklungsgesellschaft mbH, 52074 Aachen (DE)
(72) Inventor: SKRZYPCZAK, Dennis, 52074 Aachen (DE); DECKE, Robert, 52074 Aachen (DE); LIEBING, Reiner, 52074 Aachen (DE)
(74) Representative: Klunker IP Patentanwälte PartG mbB

(56) References cited:
- WO-A1-2016/185473
- US-A1- 2016 303 299

## Description

### BACKGROUND OF THE INVENTION

This invention relates to an intravascular blood pump, in particular a percutaneously insertable blood pump, for supporting blood circulation in humans or optionally also in animals and further relates to a method for producing the intravascular blood pump. For instance, the blood pump may be designed to be inserted percutaneously into a femoral artery of a patient and guided through the patient's vascular system in order, for example, to support or replace the pumping action of the heart.

While the present invention will be described in the context of an intravascular blood pump having an expandable housing, in which an expandable rotor is housed driven by an extracorporeal motor via a long and flexible drive shaft, the present invention is also applicable in other types of intravascular blood pumps.

A blood pump of the aforementioned expandable type is known, e.g., from
US 2013/0303969 A1, which discloses a catheter pump assembly. An expandable housing is located at a distal end of the catheter. The expandable housing surrounds an expandable rotor driven by a flexible drive shaft, which extends through a first lumen of the catheter. The distal portion of the catheter pump assembly may be placed inside the heart via a percutaneous access using the Seldinger technique, for example. The drive shaft contains a central lumen, which allows a guide wire together with its guide to be passed through the drive shaft to enable an exact positioning of the catheter pump assembly inside the heart. The rotor is rotatably supported in a proximal bearing arranged at the end of the catheter and proximally of the rotor. Herein, "proximal" and "distal" are seen relative to the physician. Thus, proximal designates something which is relatively close to the physician whereas distal designates something which is relatively far away from the physician when the catheter is placed.

During insertion of an intravascular blood pump, it is advantageous for the catheter to be as small as possible as this allows for a better maneuverability of the intravascular blood pump through the patient's vasculature. Accordingly, there is a need to reduce the size of the outer diameter of the catheter of an intravascular blood pump.

Further background art can be found e.g. in US 2016/303299 A1 and WO 2016/185473 A1.

### SUMMARY OF THE INVENTION

The invention is set out in the appended claims.

According to a first aspect of the invention, an intravascular blood pump comprises a catheter and a housing in which a rotor is housed, the housing being attached to the distal end of the catheter. Furthermore, in the intravascular blood pump disclosed herein, a flexible drive shaft extends through the catheter and is connected to the rotor, said drive shaft comprising at least one outer layer and at least one inner layer. The drive shaft is rotatably supported in a proximal bearing located proximally of the rotor. The proximal bearing is preferably located inside a distal end region of the catheter and/or inside a proximal end region of the housing. Alternatively, the proximal bearing, preferably the axial proximal bearing, may be located anywhere in the catheter, and there may even be more than one proximal bearing, the term "proximal" meaning here that the bearing is located anywhere proximally of the rotor. The at least one outer layer of the drive shaft is absent or thinned at a location, where the drive shaft is supported in the proximal bearing.

Preferably, the axial length of the location, where the at least one outer layer is absent or thinned is between 1 and 15 times, preferably between 2 and 5 times, the diameter of the drive shaft in the thinned section, such as between 2 mm and 5 mm. The at least one outer layer and the at least one inner layer are made from a flexible material, preferably from metal. In some embodiments, at least one of the inner layers may be a wire or a cable.

The drive shaft preferably extends through the entire catheter. The drive shaft is preferably hollow. The drive shaft preferably consists of or comprises a flexible cable which is preferably formed of differently oriented fiber layers. In particular, the drive shaft is most preferably made up of a plurality of coaxial windings, preferably with different winding directions, particularly preferably with alternating winding directions, running helically around a lumen extending axially along the drive shaft. For example, the drive shaft can comprise two coaxial windings, with opposite winding directions, and an outer diameter of the drive shaft may be between 0.4 mm and 2 mm, preferably between 0.6 mm and 1.2 mm, particularly preferably between 0.8 mm and 1.0 mm. The proximal end of the drive shaft is preferably attached to the extracorporeal electric motor. The drive shaft serves to transfer a torque from the electric motor to the rotor at the distal end of the drive shaft. In some cases, the drive shaft may comprise a stiff, rigid shaft at its distal end, onto which the rotor is attached inside the housing, in order to provide stability to the rotor.

In some embodiments, the at least one outer layer and/or at least one of the inner layers comprise or consist of wire which is wound in a winding or several windings. Each wire may comprise one strand or several strands, e.g. that may be twisted. Instead of the wire being wound in the layer, the wire may be braided, e.g., similar to the outer sheath of a kernmantle rope. The windings of one layer may form one helix. Alternatively, some or all layers may each consist of two or more helices which are preferably shifted axially, similar to a multi-start thread. Different layers may have a different handedness of the helices, e.g., an alternating handedness from one layer to the next, adjacent layer. The wire may consist of metal or may comprise metal and additional materials, such as a surface coating.

Preferably, the drive shaft is at least partly filled with a sealant penetrating into the at least one inner layer. More preferably, the sealant penetrates into all layers, i.e., the at least one outer layer and the at least one inner layer. In this, a sealant is a substance which may penetrate the layers as a fluid and then sufficiently hardens to prevent penetration of purge fluid through the respective layers. If the layers contain holes or are made of wire, sealant may penetrate across layers. In some cases, the layers, in particular the wire or wires of the drive shaft, may be partly or entirely filled with the sealant. Examples of sealants in the sense of the invention are adhesives, polymers and/or thermoplastics.

For example, the proximal bearing is configured to be purged with a purge fluid. A purge fluid may reduce friction and transport frictional heat away from the proximal bearing. Also, it may prevent blood from entering through the bearing gap. If purge fluid can flow through the drive shaft in addition to the bearing gap on its way through the proximal bearing, it is difficult to create a defined purge fluid flow. Therefore, if the drive shaft is filled with a sealant, then purge fluid flow through the drive shaft may be prevented and the creation of a defined purge fluid flow through the proximal bearing may be facilitated.

Preferably, at the location where the at least one outer layer of the drive shaft is absent or thinned, a bearing sleeve surrounds the at least one inner layer or the thinned at least one outer layer and said bearing sleeve forms an inner surface of the proximal bearing. The bearing sleeve may be a hollow cylinder surrounding the at least one inner layer or the thinned at least one outer layer. If the drive shaft is filled with the sealant inside the bearing sleeve, a purge fluid flow through the bearing sleeve may advantageously be prevented. In some cases, however, drive shafts which possess several layers may be very difficult to fill entirely with sealant. A residual purge fluid flow through the drive shaft may result. In some embodiments, there is only one inner layer and the inner layer as well as the at least one outer layer consist of wound wire. At the same time, the at least one outer layer may be completely removed in the location of the sleeve. In a design with more than two outer layers, it is possible to remove one outer layer or a plurality of outer layers so that only the at least one inner layer remains. In this case, filling the at least one inner layer inside the bearing sleeve with the sealant may be particularly effective in stopping flow through the drive shaft. This is because sealant does not have to penetrate into spaces between layers to completely seal the inside of the bearing sleeve. The bearing sleeve may advantageously provide a smooth inner bearing surface in the proximal bearing.

In another embodiment, an internal diameter of the bearing sleeve is approximately equal to an outer diameter of the at least one inner layer or to an outer diameter of the thinned at least one outer layer of the drive shaft. An outer diameter of the bearing sleeve is approximately equal to an outer diameter of the at least one outer layer of the drive shaft and may preferably be slightly greater than the at least one outer layer of the drive shaft in order to facilitate the assembly of the device. This way, the inner radial bearing surface of the proximal bearing corresponds to the outer diameter of the drive shaft and may preferably be slightly greater than the at least one outer layer of the drive shaft in order to facilitate the assembly of the device.

The at least one inner layer may be axially disconnected inside the bearing sleeve. If the at least one inner layer is axially disconnected inside the bearing sleeve, it may be particularly easy to fix the bearing sleeve on top of the at least one inner layer. Without an axially disconnected inner layer, the at least one inner layer may have to be fed through the bearing sleeve before the next following section of the at least one outer layer can be mounted on the at least one inner layer. This is particularly advantageous in embodiments in which the at least one inner layer extends distally beyond the proximal bearing.

In some embodiments, the internal space of the bearing sleeve is hydraulically separated, i.e., liquid may not flow internally from one side of the bearing sleeve to the other. In some embodiments, the bearing sleeve may not be a cylinder containing a through-hole, but a cylinder with a wall separating two blind holes. The two ends of the axially disconnected at least one inner layer may then be inserted each into a respective one of the blind holes. The wall between the holes prevents any purge fluid flow through the bearing sleeve, such that the axially disconnected at least one inner layer needs not be sealed, but merely attached to the bearing sleeve.

Preferably, the bearing sleeve is fixedly connected to the at least one inner layer or the thinned at least one outer layer of the drive shaft. T at least one he bearing sleeve may be crimped, soldered, welded, glued or shrunk to the at least one inner layer or to the thinned at least one outer layer. Gluing the bearing sleeve may advantageously avoid any distortion or warping of the bearing sleeve.

The proximal bearing preferably comprises the bearing sleeve and an outer bearing ring, the bearing sleeve being rotatably supported in the outer bearing ring. The bearing sleeve and the outer bearing ring, thus, form a radial bearing.

Preferably, the radial bearing gap between the outer bearing ring and the bearing sleeve is between 1 µm and 10 µm, more preferably between 2 µm and 8 µm wide. Most preferably, the said radial bearing gap is approximately 3.5 µm wide. Purge fluid may be pushed through the radial bearing gap of the proximal bearing. If the radial bearing gap is configured in this way, the purge fluid may flow at a reproducible speed given a suitable purge fluid pressure.

Preferably, the bearing sleeve and/or the outer bearing ring comprises one or more ceramic and/or metal. The metal is preferably MP35, 35NLT, Nitinol or stainless steel. In the case of metal, the bearing sleeve and/or the outer bearing may comprise a coating, preferably a hard coating, for example a DLC coating. Advantageously, the bearing sleeve and/or outer bearing ring designed in this manner allow for a lightweight, durable proximal bearing.

In a preferred embodiment, one or more protective rings may be provided extending over both the at least one outer layer of the drive shaft and a respective portion of the bearing sleeve. Thereby, the risk that the drive shaft breaks due to a change of stiffness at the transition between the at least one inner layer of the drive shaft with a relatively small diameter and the at least one outer layer of the drive shaft with a relatively large diameter is effectively reduced. Preferably, a distal protective ring is provided to extend over the drive shaft and a distal portion of the bearing sleeve, and a proximal protective ring is provided to extend over the drive shaft and a proximal portion of the bearing sleeve. Alternatively, the bearing sleeve and one protective ring may form a single integral member.

The proximal bearing with the bearing sleeve and the protective rings may preferably be assembled as follows. The at least one inner layer and the at least one outer layer of the drive shaft may be mechanically separated from each other or may be mounted during production, such that the at least one inner layer protrudes from the at least one outer layer. After mechanical separation of the at least one outer layer from the at least one inner layer, the at least one outer layer is pulled off of the at least one inner layer while turning slightly. On the at least one outer layer of the drive shaft, a first protective ring is placed, such that a longer part thereof is fixed to the at least one outer layer of the drive shaft mechanically or in another way. A shorter part of the first protective ring overlaps with the at least one inner layer of the drive shaft. Thereafter, a low-viscosity adhesive is brought into the overlapping area and used to glue the bearing sleeve onto the at least one inner layer of the drive shaft. The bearing sleeve is positioned such that it reaches into and overlaps with the first protective ring. After the adhesive is cured, the combination of drive shaft, first protective ring and bearing sleeve may be tested for liquid impermeability. The outer bearing ring is then positioned onto the bearing sleeve. The previously removed at least one outer layer is pushed onto the at least one inner layer until it touches the bearing sleeve and is glued into position. Then, a second protective ring is placed on top of the previously removed at least one outer layer and overlaps with the bearing sleeve. The second protective ring is positioned such that a predetermined axial play between the outer bearing ring and the bearing sleeve is set. The overlapping of the bearing sleeve with the protective rings may cause radial stabilization of the bearing sleeve and may lead to a prevention of a sharp change in stiffness at the end of the bearing sleeve. The second protective ring is then fixed to the drive shaft mechanically or in another way. Fixation of the protective rings onto the at least one outer layer may additionally prevent loosening of the at least one outer layer. The protective rings preferably have an inner diameter at least as wide as the outer diameter of the bearing sleeve. The protective rings preferably surround the end of the at least one outer layer adjacent to the bearing sleeve.

In some embodiments, the proximal protective ring overlapping with the bearing sleeve may be axially placed between the outer bearing ring and a restriction member. The restriction member acts as a limit restricting the axial movement of the drive shaft relative to the outer bearing ring. In one embodiment, the rotor or a rotor shaft mounted distally of the outer bearing ring may form the distal protective ring, in which case the restriction member advantageously prevents the touching of the rotor or rotor shaft and the outer bearing ring.

As mentioned above, the proximal bearing is preferably located inside a distal end region of the catheter and/or a proximal end region of the housing. The outer bearing ring is preferably fixed to the catheter and/or to the proximal section of the housing. If the proximal bearing is purged, purge fluid may exit the catheter via the bearing gap of the proximal bearing. The inner diameter of the outer bearing ring of the proximal bearing is preferably between 0.6 mm and 2.2 mm, more preferably between 0.9 mm and 1.3 mm. The bearing gap of the proximal bearing is preferably between 1 µm and 10 µm, more preferably between 2 µm and 8 µm.

Preferably, the protective ring is fixedly connected to the drive shaft. The protective ring is preferably crimped, soldered, welded, glued or shrunk to the at least one outer layer and/or to the bearing sleeve.

Preferably, the protective ring comprises one or more ceramic and/or metal, in particular MP35, 35NLT, Nitinol or stainless steel. In the case of metal, the protective ring may be hard coated, for example DLC-coated.

Preferably, at least the proximal protective ring provides an axial bearing surface in the proximal bearing. The surface of the proximal protective ring facing the outer bearing ring preferably forms an axial bearing with an opposing surface of the outer bearing ring. The distal as well as the proximal protective ring preferably forms a stop element for the outer bearing ring to prevent the outer bearing ring from sliding off of the bearing sleeve.

Preferably, two different adhesives are used on the drive shaft. A first adhesive is preferably used to penetrate the at least one outer and/or the at least one inner layer, in particular the outer and/or the inner windings thereof. The first adhesive may be the sealant. The first adhesive preferably has a particularly low viscosity to be able to penetrate the outer and/or the inner windings completely. The first adhesive preferably has a viscosity in the range from 80 cPs to 200 cPs before hardening. A suitable adhesive is a two-components epoxy resin. A second adhesive is preferably used to connect the sleeve and/or the at least one protective ring to the drive shaft. Preferably, the first adhesive has a lower viscosity than the second adhesive. The second adhesive preferably has a medium or paste-like viscosity. A suitable adhesive is a two-components epoxy resin.

According to a particularly preferred embodiment, the bearing sleeve extends into the rotor. Frequently, the bearing sleeve is stiffer than the drive shaft such that the rotor may have more rigid support in comparison to other embodiments in which the rotor is mounted on the distal end of the drive shaft.

Preferably, the radial bearing gap between the outer bearing ring and the bearing sleeve is between 1 µm and 10 µm, more preferably between 2 µm and 8 µm wide, most preferably approximately 3.5 µm wide.

The intravascular blood pump may further comprise a distal bearing for rotatably supporting a distal end of the rotor. The distal bearing is located either inside or distally of the rotor. Preferably, the distal bearing comprises a static support member which protrudes into or up against the distal end of the rotor. Alternatively, a distal end of the drive shaft or of the bearing sleeve may be supported by the distal bearing.

In another embodiment, the drive shaft is not supported in the distal bearing. Instead, the rotor may be mounted on or to the very end of the drive shaft or on a distal extension of the proximal bearing sleeve, respectively, such that it is the distal end of the rotor which is supported by the static support member extending into or up against the rotor. In this way, tendinous structures are less likely to be caught by rotating parts, in particular if no rotating structure extends beyond the leading edge of the rotating blade. This may lead to a safer intravascular blood pump with a longer lifetime.

Preferably, the bearing sleeve comprises a proximal portion located proximally of the outer bearing ring and forming an axial bearing with a proximal surface of the outer bearing ring. The bearing sleeve preferably comprises a distal portion extending from the proximal portion of the sleeve distally into the outer bearing ring, wherein the distal portion of the bearing sleeve forms a radial bearing with the outer bearing ring. The described design of the proximal bearing may advantageously achieve low friction and high durability, in particular when a purge fluid is provided to flow through the radial bearing.

A bearing sleeve of this particular structure is particularly useful for a proximal bearing which is placed next to the rotor rather than somewhere deep inside the catheter. In this case, the outer bearing ring is located, in particular fixedly connected, inside the distal end region of the catheter or inside the proximal end region of the housing. The outer bearing ring may be pressfitted and/or glued into the catheter and/or housing. In some embodiments, the outer bearing ring may be fitted to both the housing and the catheter, thereby connecting the housing with the catheter. A proximal bearing located at the distal end of the catheter or at the proximal end of the housing may provide particularly stable support for the drive shaft and rotor.

The restriction member mentioned above may be located proximally of the bearing sleeve inside the catheter and/or the housing to prevent the bearing sleeve from slipping out of the outer bearing ring. Preferably, an inner diameter of the restriction member is slightly larger than the diameter of the flexible drive cable to avoid frictional contact therewith and to allow purge fluid to pass through.

Preferably, the proximal end region of the housing and/or the distal end region of the catheter contain one or more radial through-holes. The radial through-holes may increase elasticity to allow press-fitting of the proximal bearing into the housing and/or catheter. The through-holes further allow the introduction of glue and position monitoring when inserting the proximal bearing. Notably, the glue is used to seal the gap between the bearing and the housing to avoid leakage of purge fluid, i.e. care should be taken that the gap is filled completely. The radial through-holes may have a diameter of between 0.5 mm and 1 mm. Elongated holes with an elongation in the circumferential direction can be advantageous for filling the notch of the bearing, in which case the afore-mentioned diameter refers to the smaller diameter of the elongated hole.

Preferably, the flexible drive shaft contains a reinforcement element extending longitudinally within a central lumen of the drive shaft, preferably a coaxial stiff reinforcement rod. More specifically, the drive shaft is in some embodiments reinforced by a reinforcement element at its distal end region. This is particularly advantageous when the drive shaft extends into and in some embodiments up to the distal end of the rotor. Accordingly, the reinforcement element may extend from an area proximally of the proximal bearing to the distal end of the drive shaft. The reinforcement element is preferably a metal rod, for example made of spring steel, a metal wire or a carbon wire. In one embodiment the metal wire is made of 1.4310 stainless steel.

The bearing sleeve comprises a portion extending distally of the outer bearing ring and the rotor may be mounted on the said portion extending distally of the outer bearing ring. Such a design may allow for a particularly stable configuration of the rotor. In particular, the bearing sleeve may extend along a major part of the axial length of the rotor, more preferably up to the distal end of the rotor.

The rotor is preferably at a distance of between 0.001 mm and 8 mm from the distal surface of the outer bearing ring. A minimum distance between the rotor and the proximal bearing is desirable as this prevents the rotor from being caught in the proximal bearing.

The intravascular blood pump is preferably designed as an expandable blood pump with a housing having an expandable section. In some embodiments, the housing comprises or consists of a shape-memory material, in particular Nitinol. The diameter of the percutaneously insertable blood pump is generally limited by the internal diameter of the smallest blood vessel to be traversed. The intravascular blood pump may be moved through blood vessels with the housing in its collapsed state. On reaching the heart or larger vessels, the housing of the intravascular blood pump may be expanded. This allows the percutaneous insertion of a larger blood pump into the heart than otherwise possible. With such a larger blood pump, it may be possible to generate higher blood flow rates.

When the blood pump is designed as an expandable pump, a cannula is preferably provided around a portion of the drive shaft, which lies in the vicinity of the rotor, and the housing and the rotor are configured to be transferred at least in part into the cannula. During such a transfer, the expandable section of the housing and the rotor are compressed at least along a radial direction extending transversely to a longitudinal direction, from an expanded state to a compressed state. Preferably, parts of the rotor, such as the rotor blades, or the entire rotor, are also expandable to allow a larger rotor to be inserted into the heart, which may improve flow rates.

In some embodiments, the static support member of the distal bearing protrudes up against the distal end of the rotor. In comparison to embodiments in which the static support member protrudes into the rotor, a particularly flexible pump section of the intravascular blood pump may be created. High flexibility of the pumping device is specifically advantageous during insertion and removal of the intravascular blood pump. If the static support member does not protrude into the rotor and is instead merely placed up against the distal end of the rotor, it may intentionally dislodge from the rotor when the pump section is bent during maneuvering of the pumping device through the blood vessels. When the pump section reaches its final destination inside the heart, it may straighten and the static support member may resume a position in which it protrudes up against the distal end of the rotor.

Preferably, the static support member is attached to the distal end of the housing, wherein the expansion of the housing may provide an axial force via the static support member onto the distal end of the rotor. Preferably, the force is equal to or less than 1.8 N. When the static support member protrudes up against the distal end of the rotor, it may limit further expansion of the housing.

When the housing is compressed, the static support member preferably moves away from the distal end of the rotor. In this state, the pump section is more flexible as relative radial movement of the static support member and the rotor becomes possible. This may be advantageous during insertion of the intravascular blood pump or during retrieval.

In certain embodiments, the intravascular blood pump comprises a nose at the distal end of the rotor. When the housing is in its expanded state, the nose protrudes into the static support member, which preferably possesses a correspondingly formed recess. The nose has the purpose to center the rotation of the rotor and to bring the rotor and the static support member into a correct relative position after expansion of the housing. The nose preferably protrudes over the surrounding surface of the rotor by between 0.1 mm and 2 mm, more preferably between 0.2 mm and 1 mm and most preferably between 0.3 mm and 0.5 mm. The depth of the recess in the static support member corresponds to the nose and is preferably between 0.1 mm and 2 mm, more preferably between 0.2 mm and 1 mm and most preferably between 0.3 mm and 0.5 mm.

In some embodiments in which the static support member protrudes into the rotor, the rotor comprises an axial stop for the static support member, such as a recess at its distal end having a bottom or a step. The bottom or step defines an axial stop for a proximal end of the static support member in the distal end of the rotor. This is particularly advantageous in the context of an expandable blood pump. In its expanded state, the proximal end of the static support member, which protrudes axially into the rotor, may be in contact with the axial stop, thereby preventing further expansion of the housing and, thus, limiting a radial gap width between an outer edge of the rotor blades and an inner surface of the expandable housing. Alternatively, in the expanded state of the expandable blood pump, the proximal end of the static support member and the axial stop may form a gap, which is preferably between 0.01 mm and 1 mm, more preferably between 0.01 mm and 0.1 and most preferably between 0.01 mm and 0.05 mm wide in an axial direction.

A length of the recess at the distal end of the rotor, which is measured in an axial direction, may for instance be between 0.5 mm and 8 mm, preferably between 1 mm and 5 mm, particularly preferably between 1.5 mm and 2.5 mm. When the housing is moved into the cannula, the housing preferably stretches by between 0.5 mm and 2.5 mm axially, more preferably between 1 mm and 2 mm, most preferably by approximately 1.7 mm.

Inside the distal end of the drive shaft, i.e., inside the rotor shaft, the intravascular blood pump may contain an optional fluid line arranged to guide a purge fluid through the rotor to the distal bearing. In some embodiments, the rotor comprises a hollow section as a part of the fluid line, wherein the intravascular blood pump is arranged to guide the purge fluid through the hollow section of the rotor to the distal bearing. The purge fluid may be transported to the fluid line via the catheter. The purge fluid may enter the catheter and/or the drive shaft inside a housing of the electric motor. The purge fluid may flow inside the catheter adjacent to the drive shaft. Where the drive shaft is hollow, purge fluid may flow partly, predominantly or entirely through the drive shaft lumen. From the distal end of the catheter to the rotor, the purge fluid may flow through the drive shaft. At least in the space between the distal end of the catheter and the proximal end of the rotor, the drive shaft may comprise a cover to avoid the purge fluid from leaking from said space.

Alternatively, the purge fluid may not be guided through the main lumen of the catheter, which contains the drive shaft, but through one or more separate, secondary lumina.

At a distal end region of the catheter, the purge fluid preferably transfers into the fluid line inside the rotor shaft. In some cases, the rotor shaft or the rotor hub may have a central lumen to accommodate the fluid line. In particular, in the case of a hollow drive shaft, the drive shaft may extend into the rotor to form both the rotor shaft and the fluid line, or the hollow drive shaft may be extended by a hollow tube to form both the rotor shaft and the fluid line. Alternatively, a distal extension of the bearing sleeve of the proximal bearing may form the hollow drive shaft. The hollow drive shaft may in some locations be permeable to purge fluid.

In a purged proximal and/or distal bearing, blood is less likely to enter the bearing gap. As a result, blood clots are prevented. In addition, a purged bearing may have less friction than the alternative bearings in the prior art. In particular, the purge fluid lubricates the bearing and can transport frictional heat away from the bearing. This may allow higher rotational speeds, lower power consumption and an increased lifetime of the blood pump. The purge fluid may be any biocompatible fluid suitable for purging the bearing. Examples of a suitable medical fluid include saline solution, glucose solution and/or water, each of them with or without heparin.

In alternative embodiments, the proximal and/or distal bearing is not purged. Accordingly, there is no transport of purge fluid to the proximal and/or distal bearing and the intravascular blood pump may not comprise a fluid line.

The distal bearing is preferably arranged such that the purge fluid may exit between the static support member and the distal end of the rotor, into which or up against which the static support member protrudes. Preferably, the distal bearing is arranged such that the purge fluid flows from a distal end of the fluid line to the distal bearing. In particular, the intravascular blood pump may be arranged such that any purge fluid passing through the hollow drive shaft or rotor shaft exits entirely or at least in part through the distal bearing. By applying a suitable pressure, the purge fluid may be urged through the bearing gap of the distal bearing, which is in some embodiments the gap bounded by the static support member and the adjacent section of the rotor. Preferably, the pressure of the purge fluid is in a range of 300 mmHg (0.4 bar) to 1500 mmHg (2 bar), more preferably in a range of 600 mmHg (0.8 bar) to 1100 mmHg (approx. 1.5 bar). If the distal bearing is purged and the rotor comprises a nose that protrudes into the static support member, the nose may contain at least one opening to allow the purge fluid to enter the bearing gap between the nose and the static support member.

In some embodiments, a distal end of the static support member is mounted at a distal end of the housing. The distal end of the housing may provide stable support for the static support member which supports the distal end of the rotor.

The static support member preferably comprises a pin extending from distally to proximally and protruding up against or, preferably, into the distal end of the rotor. Thus, the pin may be arranged to form the distal bearing for the rotor. In embodiments in which the distal bearing is purged, the pin is preferably arranged such that purge fluid may exit between the pin and the rotor mounted on the pin.

Preferably, the pin possesses a circular cross-section. However, other cross-sections are equally possible in the distal part of the pin which is located outside the rotor. For example, the pin may have an oval cross-section. In some embodiments the pin may be hollow. Alternatively, the pin may be made of solid material. Preferably, the pin is tapered towards its proximal end. The pin may be elastically bendable, preferably such that during bending of the pump head, the rotor keeps concentric to the housing.

Preferably, an inner diameter at the distal end of the rotor, into which the static support member, in particular the pin, axially protrudes, is between 0.3 mm and 1.5 mm, more preferably between 0.5 mm and 1.2 mm and most preferably between 0.7 mm and 0.9 mm wide. Preferably, the radial bearing gap between the outside of the pin and its opposite bearing surface is between 1 µm and 10 µm, more preferably between 2 µm and 8 µm wide.

In some embodiments, the pin is particularly long and protrudes into the rotor and extends proximally through the entire rotor. Preferably, the pin exits the rotor proximally and continues inside the drive shaft, e.g., ending inside the proximal bearing. In this case, the end of the pin may be arranged inside the portion of the drive shaft that is situated in the proximal bearing. By employing such a long pin that extends through the entire length of the rotor and into the proximal bearing, a particularly stiff and low-vibration pump may be created. Alternatively, the pin may extend even further to a point proximally of the proximal bearing. The pin extending through the rotor may be purged or unpurged and may be used in conjunction with a hollow drive shaft or with a drive shaft that is only hollow along a part of its length.

Preferably, the material of the pin comprises at least one of the following materials: A biocompatible material, in particular one or more of MP35N, 35NLT, Nitinol, stainless steel (in particular medical-grade stainless steel) and ceramics. The surface of the pin may comprise a coating, for example a hard coating, for example a diamond-like carbon (DLC) coating.

Preferably, the length of the pin by which the pin protrudes into the distal end of the rotor during an operational state of the intravascular blood pump is between 0.5 mm and 8 mm, preferably between 1 mm and 5 mm, particularly preferably between 1.5 mm and 2.5 mm. The longer the internal length is, the stiffer the rotor support is and, thus, the better controllable is the width of the gap between the outer edge of the rotor blades and the inner surface of the housing. The blades must not touch the inner surface of the housing, and the gap should be sufficiently large to prevent blood damage. A stiffer supported rotor can also be operated with lower excursions and less vibration, which improves hemocompatibility.

The pin may have a sufficient length to remain within the distal end of the rotor when the housing and the rotor are in the compressed state. The length of the pin, which remains inside the distal end of the rotor when the housing and the rotor are in the compressed state, is preferably more than 1.5 mm, more preferably more than 1.7 mm and most preferably more than 2 mm. When the housing and the rotor are compressed before deployment of the blood pump, the housing is extended in a longitudinal direction and the static support member, in particular the pin, extending into the distal end of the housing may possibly move out of the rotor entirely. Then, when the housing is expanded again, the pin might not move back into the rotor and the pump may not be functional. Therefore, if the pin is chosen with a sufficient length such that the pin stays inside the rotor even in the compressed state of the housing, such a problem may be avoided.

In embodiments with a pin, the distal bearing surfaces are the surface of the pin as well as a distal outer bearing surface, which may be provided by the rotor itself or by a sleeve of the distal bearing in the hub of the rotor. In some cases, the distal outer bearing surface may be provided by the stiffening element mentioned above.

The sleeve of the distal bearing may have an inner diameter preferably ranging from 0.3 mm to 1.5 mm, more preferably from 0.5 mm to 1.2 mm and most preferably from 0.7 mm to 0.9 mm.

In some embodiments, the intravascular blood pump comprises a flexible atraumatic tip to avoid damage to the patient's tissue. The atraumatic tip can be made of a flexible medical-grade polymer such as Pebax^{®} or Polyurethane. Preferably, the flexible atraumatic tip is designed as a pigtail or in a J-form.

The intravascular blood pump described above can be used in a patient, that is, it is inserted and operated inside the patient to support blood flow.

### BRIEF DESCRIPTION OF THE DRAWINGS

Hereinafter the invention will be explained by way of example with reference to the accompanying drawings. The accompanying drawings are not drawn to scale. In the drawings, identical or corresponding components illustrated in various figures are represented by the same numeral. For purposes of clarity, not every component may be labeled in every drawing. In the drawings:
Fig. 1 is a schematic representation of an intravascular blood pump, which is positioned within the left ventricle of the heart;
Fig. 2 shows a schematic representation of an intravascular blood pump;
Figs. 3A and 3B show schematic representations of an intravascular blood pump in an expanded and a compressed state;
Figs. 4A, 4B and 4C show a schematic representation of an intravascular blood pump with a static support member extending into the distal end of the rotor according to a first embodiment;
Fig. 5 shows a schematic representation of an intravascular blood pump with a static support member extending into the distal end of the rotor according to a second embodiment;
Figs. 6A to 6D show schematic representations of an intravascular blood pump with a rotor having a nose at its distal end according to a third embodiment;
Fig. 7 shows a schematic representation of an intravascular blood pump with a proximal and a distal bearing;
Figs. 8A and 8B show a schematic representation of the path of purge fluid in an intravascular blood pump;
Fig. 9A shows a drive shaft comprising an outer layer and an inner layer;
Fig. 9B shows a drive shaft with a bearing sleeve, an outer bearing ring and protective rings;
Fig. 10A shows a hydraulically divided bearing sleeve;
Fig. 10B shows a bearing with a restriction member;
Figs. 11A and 11B show bearings with restriction members and a rotor;
Figs. 12A and 12B show two different embodiments of a proximal bearing with an outer bearing ring and a specifically formed bearing sleeve; and
Figs. 13A to 13D show hydrodynamic axial bearings.

### DETAILED DESCRIPTION OF THE DRAWINGS

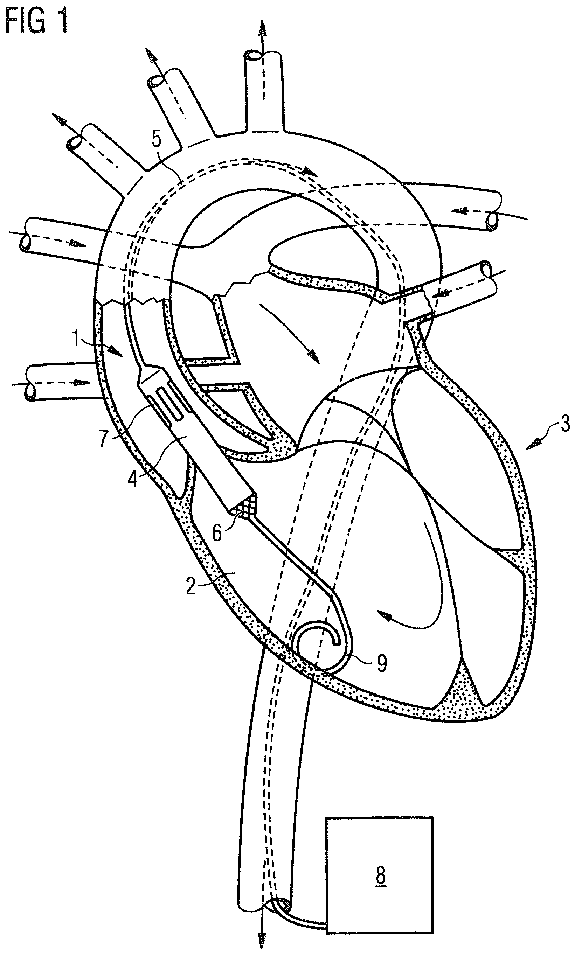

Fig. 1 shows the use of an intravascular blood pump 1 for supporting, in this particular example, a left ventricle 2 of a human heart. The intravascular blood pump 1 comprises a catheter 5 and a pumping device, the pumping device comprising a pump section 4 mounted at a distal end region of the catheter 5. The intravascular blood pump 1 may be placed inside the heart using a percutaneous, transluminal technique. For example, the intravascular blood pump 1 may be introduced through a femoral artery. However, alternative vascular access is equally possible, such as access through the subclavian artery. After passing through the femoral artery, the catheter 5 may be pushed into the aorta such that the pump section 4 reaches through the aortic valve into the heart. The positioning of the pump section 4 in Fig. 1 serves purely as an example, whereas different placements are possible, such as positioning the pump section 4 inside the right ventricle of the heart.

The pump section 4 comprises a rotor 10 to cause blood to flow from a blood flow inlet 6 at a distal end of the pump section 4 to a blood flow outlet 7 located proximally of the blood flow inlet 6. The catheter 5 houses a drive shaft 12 driven by the electric motor 8, which is preferably placed outside the patient's body. The drive shaft 12 drives the rotor contained inside the pump section 4. At its distal end, the pump section 4 possesses a flexible atraumatic tip 9 having the form of a pigtail or a J-form, which facilitates placement of the intravascular blood pump 1 by aiding navigation inside the patient's vascular system. Furthermore, the softness of the flexible atraumatic tip 9 allows the pump section 4 to support itself atraumatically against the wall of the left ventricle 2.

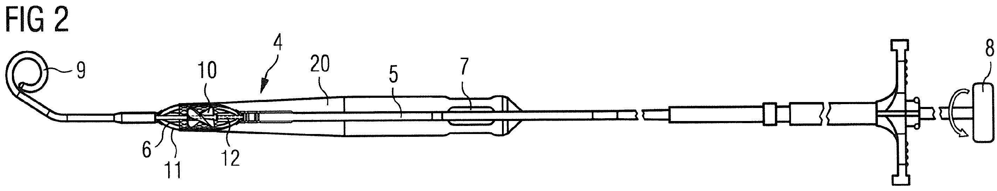

Fig. 2 shows the intravascular blood pump 1 in further detail. The rotor 10 is located inside a housing 11. In this embodiment, both the rotor 10 and the housing 11 are compressible. In this case, the intravascular blood pump 1 is transported through the patient's vascular system while both the rotor 10 and the housing 11 are in their compressed state. Once the pump section 4 is at its target location, the housing 11 and rotor 10 are expanded. The flexible atraumatic tip 9 is situated at the distal end of the housing 11. The drive shaft 12 is realized as a drive shaft cable. The drive shaft 12 with the rotor 10 arranged at a distal end thereof can be seen protruding from the distal end of the catheter 5. When the rotor 10 inside the housing 11 is rotated by means of the drive shaft 12, blood is drawn into the blood flow inlet 6 at the distal end of the housing 11 and through the housing 11 into a downstream tubing 20, which is attached to the housing 11 and extends proximally. The blood is then ejected from the downstream tubing 20 into the aorta through the more proximally located blood flow outlet 7 provided in the downstream tubing 20, the blood flow outlet comprising a plurality of outlet openings. The downstream tubing 20 is made of a flexible material such that it can be compressed by the aortic valve as the patient's heart is pumping. The downstream tubing 20 is typically expanded mainly due to the active blood flow generated by the rotor 10 during rotation. By placing the blood flow inlet 6 inside the left ventricle 2 and the blood flow outlet 7 inside the aorta, the intravascular blood pump 1 may support the patient's systemic blood circulation. If the intravascular blood pump 1 is configured and placed differently, it may be used, e.g., to support the patient's pulmonary blood circulation instead.

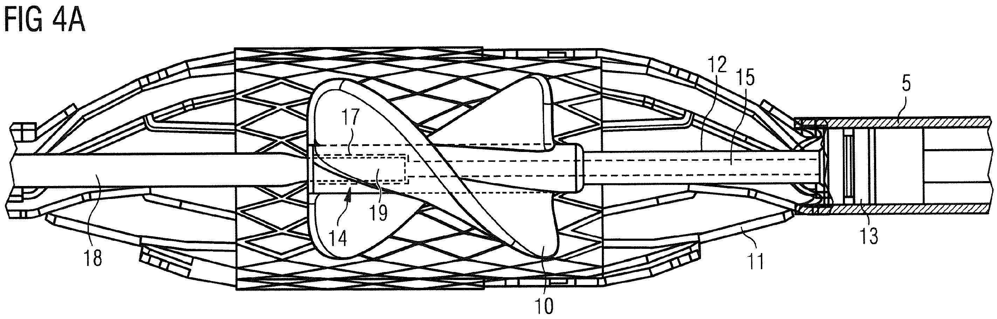

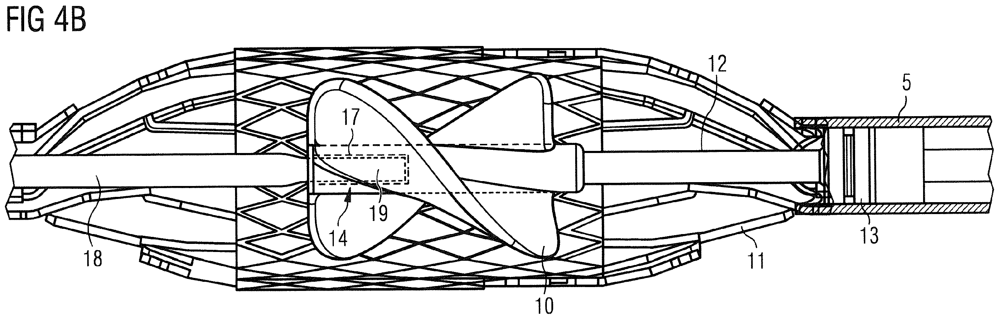

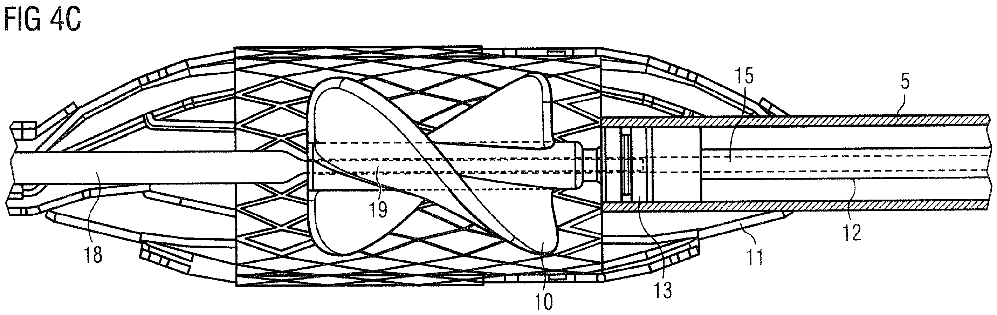

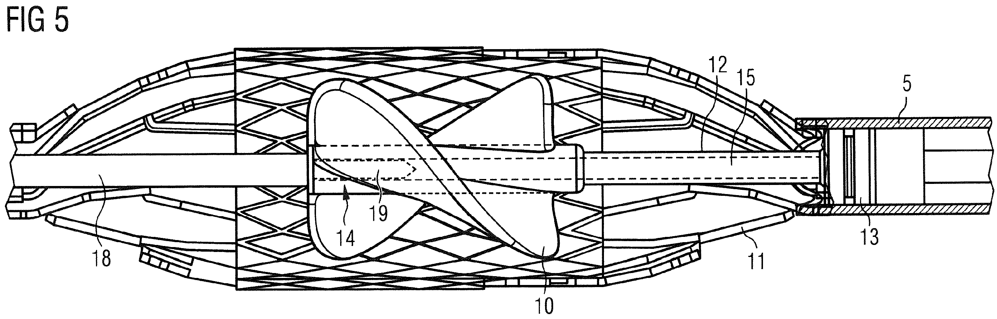

In this example, a liquid, in particular a purge fluid, is supplied from outside the patient's body through the catheter 5 to the pump section 4. Inside the pump section 4, the liquid may be used to purge one or more bearings in order to reduce friction and cool the pump section 4, as will be explained further in relation to Figs. 4 and 5. Preferably, the liquid is used to purge at least the distal bearing. In such case, the pressure of the purge fluid is chosen to be higher than the blood pressure of the patient in order to prevent blood from entering the bearing. Preferably, the pressure of the purge fluid is in a range of 300 mmHg (0.4 bar) to 1500 mmHg (2 bar), more preferably in a range of 600 mmHg (0.8 bar) to 1100 mmHg (approx. 1.5 bar).

The housing 11 is preferably produced from a shape-memory material, such as Nitinol, and provides a cage around the rotor 10. As can be seen in Fig. 5, a central part of the housing 11 carries a sleeve, which defines a channel through which blood is pumped by means of the rotor 10. Proximally and distally of this channel, the housing 11 allows blood to be sucked into the housing 11 and pushed out of the housing 11 into the downstream tubing 20 (as shown in Fig. 2).

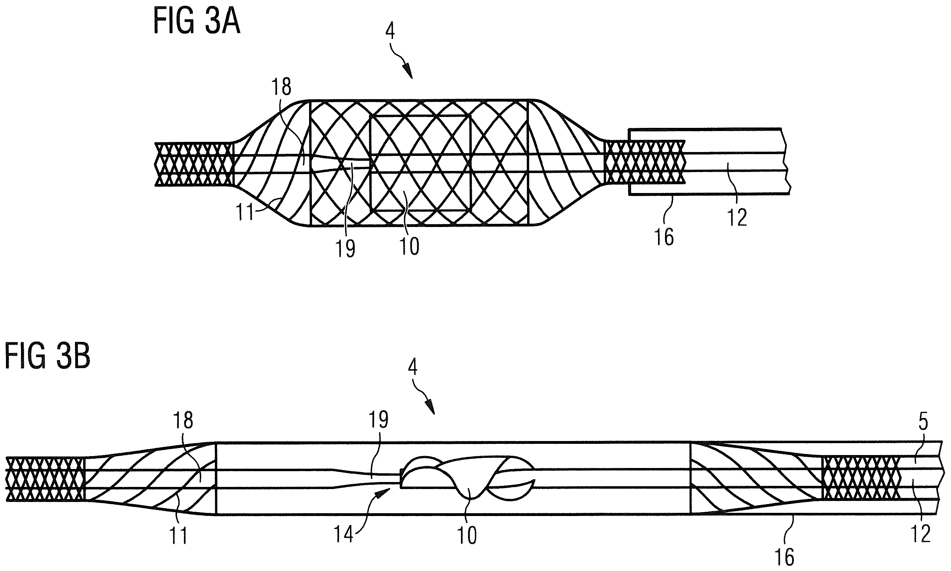

Figs. 3A and 3B show the pump section 4, its rotor 10 as well as its housing 11 in an expanded and in a compressed state, respectively. A cannula 16 is arranged at the distal end of the catheter 5. Initially, before deployment of the intravascular blood pump 1, the pump section 4 is provided in its compressed state inside the cannula 16. The cannula 16 can be a cannula 16 pertaining to the catheter 5 or a peel-away-sheath to aid the insertion of the catheter 5 into the body of a patient. When a physician has determined that the catheter 5 is placed correctly inside a patient's vascular system, he or she will push the housing 11 out of the cannula 16. With the cannula 16 removed, the housing 11 will expand due to its shape-memory properties and/or due to its hyper-elastic properties. At the same time, the rotor 10 expands due to its elasticity. As the housing 11 expands radially away from the drive shaft 12, it contracts in the longitudinal direction.

The rotor 10 is supported in a distal section of the rotor 10 by a distal bearing 14 comprising a static support member 18 with a pin 19, the static support member 18 being attached to the housing 11 at one end thereof and extending into the distal end of the rotor 10 with its pin 19 on the other end thereof so that upon the expansion of the housing 11 the pin 19 can move axially inside the distal end of the rotor 10. Preferably, the pin 19 is sufficiently long for it to remain inside the rotor 10 when the housing 11 is in its compressed state. When the intravascular blood pump 1 is in its expanded state and needs to be removed from the heart, the physician pulls the housing 11 back into the cannula 16, which will cause the housing 11 to compress radially and extend longitudinally so that the distal end of the housing 11 moves away from the rotor 10 along with the static support member 18 and its pin 19, which extends into the distal end of the rotor 10. The smaller diameter of the housing 11 thus achieved facilitates the removal of the intravascular blood pump 1 from the patient.

In prior art distal bearings 14, the drive shaft 12 sometimes extends distally of the rotor 10 and into the distal bearing. This, however, may cause tendinous chords of the heart to be entangled with the drive shaft 12 possibly leading to clotting and device failure. Therefore, the use of the static support member 18 as part of the distal bearing 14, which does not involve rotating parts distal to the rotor 10 and distal to the rotor blades, is advantageous.

Figs. 4A and 4B show the pump section 4 according to a first embodiment in further detail including the housing 11 and the rotor 10, which is driven by the drive shaft 12. The drive shaft 12 is rotatably supported both in a proximal bearing 13 at the distal end of the catheter 5 proximally of the rotor 10 (or in a proximal part of the housing) and in a distal bearing 14 located at the distal end of the rotor 10. In Fig. 4A, the drive shaft 12 is hollow at its distal end or, more specifically, the rotor shaft is hollow so as to form a fluid line 15 through which a purge fluid may be pumped towards the distal bearing 14. Where the drive shaft is hollow and extends up to the distal end of the rotor 10, the rotor 10 may be formed directly on the distal end of the drive shaft 12 so that the rotor shaft is formed by the drive shaft, whereby in the regions of the proximal and distal bearings the drive shaft 12 may be stiffened, e.g., by injection-molded plastic material, and provided with appropriate outer and inner bearing surface finishes, respectively. Alternatively, the entire end region including the bearing sections of the drive shaft 12 may be stiffened in order to obtain a stiffer structure of the pump section. For example, the drive shaft 12 is thinned at its distal end and a stiff hollow tube is slipped over the thinned end and extends distally to form the rotor shaft and bearing sections. The purge fluid may be transported through the fluid line 15 in the rotor shaft to the distal bearing 14. In the embodiment shown in Fig. 4A, the purge fluid can be urged through the central fluid line 15 to exit the drive shaft 12 at its distal end and further through a bearing gap of the distal bearing 14 into the blood stream. The purging of the distal bearing 14 by the purge fluid leads to less friction and thus to less wear on the distal bearing and, furthermore, prevents blood from entering into and clogging the bearing gap.

For the intravascular blood pump 1 to be efficient, a large rotor diameter is desirable. However, as the gap between the rotor 10 and the housing 11 gets smaller, the risk of blood cells or the rotor 10 being damaged increases. If only a proximal bearing 13 is used, the system may oscillate and the gap between the tip ends of the blades of the rotor 10 and the inner surface of the housing 11 may undergo large variations. When the flexible atraumatic tip 9 touches the heart wall, the movement of the heart can cause bending of the housing, which could lead the housing to touch the rotor. Touching of housing and rotor during use could cause a significant increase of damage to blood cells and may also cause wear with particles from the housing and/or rotor getting into the blood stream. By using both a proximal bearing 13 and a distal bearing 14, as illustrated in Figs. 4A and 4B, the position of the rotor 10 is more stable and the variation of the size of said gap is lower than with just one bearing. For a given housing 11, this may allow the rotor 10 diameter to be larger, which allows for a higher flow rate of the intravascular blood pump 1 without the housing touching the rotor.

At its distal end, the rotor 10 comprises a recess 17. The static support member 18 fixed relative to the distal end of the housing 11 protrudes with its pin 19 into the recess 17. The bottom 19 of the recess 17 in Fig. 4A is formed as a step and defines a stop inside the rotor 10 against which the pin 19 of the static support member 18 can rest. In Fig. 4A, the fluid line 15 penetrates the bottom of the recess 17 to allow purge fluid to exit the distal bearing 14 between the pin 19 and the recess 17.

The embodiment of the intravascular blood pump 1 in Fig. 4B is similar to the embodiment in Fig. 4A. Importantly, however, the distal bearing in Fig. 4B is not purged and is designed to operate in blood instead. Thus, the drive shaft 12 does not need to be hollow. Accordingly, there is no fluid line 15 in Fig. 4B. The bottom of the recess 17 does not contain an opening for purge fluid to flow through the bearing gap between the pin 19 and the recess 17. In such an embodiment, less purge fluid may be required. If the proximal bearing is not purged, the intravascular blood pump may require no purge fluid at all.

Fig. 4C shows a similar embodiment to Figs. 4A and 4B. Here, the pin 19 is particularly long and extends proximally through the rotor shaft and into the drive shaft 12. In the embodiment of Fig. 4C, the proximal end of the pin 19 is located inside the part of the drive shaft 12, which is located inside the proximal bearing 13. In alternative embodiments, the proximal end of the pin 19 may be located, e.g., proximally of the proximal bearing 13 or between rotor 10 and proximal bearing 13.

By having the pin 19 extend into the proximal bearing 13, a greater stiffness of the intravascular blood pump 1 may be achieved. Furthermore, the pin 19 shown in Fig. 4C may help to reduce vibrations of the intravascular blood pump 1 during its operation and may decrease undesired bending.

The proximal bearing 13 in Fig. 4C is located inside the housing 11, distally of the proximal bearing's 13 location in Figs. 4A and 4B. The distance between the proximal bearing 13 and the rotor 10 is particularly small in the embodiment shown, e.g., smaller than the outer diameter of the proximal bearing 13. The short distance may further increase the stiffness of the intravascular blood pump 1.

The pin 19 in Fig. 4C is combined with a hollow drive shaft 12 such that, in some embodiments, purge fluid may flow through the drive shaft 12 and past the pin 19 to exit at the distal end of the rotor 10. Alternatively, no purge fluid may be used in some embodiments. In this case, the long pin 19 of Fig. 4C may be combined with a drive shaft that is only hollow along some part of its length.

Fig. 5 shows the pump section 4 according to a second embodiment again with a compressible housing 11 and a rotor 10 driven by a hollow drive shaft 12, which is rotatably supported in a proximal bearing 13 arranged proximally of the rotor 10 at the distal end of the catheter 5. In this embodiment, the pin 19 of the static support member 18 forming part of the distal bearing 14 has a pointed end. If the dimensions of the housing 11 and the pin 19 are such that the pin 19 leaves the rotor 10 when the housing 11 is compressed, the pointed end of the pin 19 facilitates reintroduction of the pin 19 into the opening at the distal end of the rotor 10 when the housing 11 is expanded again. Preferably, the pin 19 is sufficiently long for the pin 19 to remain inside the rotor 10 when the housing 11 is in the compressed state. This may avoid the circumstance, in which the pin 19 fails to re-enter the rotor 10 when the housing 11 is being expanded. In some cases, it is not necessary for proper function that a required bearing gap is present over the full length of the pin 19. Rather, it is sufficient for the bearing gap between the outside of the pin 19 and its opposite bearing surface to be between 1 µm and 10 µm, more preferably between 2 µm and 8 µm wide in at least one location.

In this embodiment, rather than providing a bottom or a step in the opening at the distal end of the rotor 10, the static support member 18 may be provided with a shoulder against which the rotor 10 abuts in an expanded state of the housing 11, thereby limiting further expansion of the housing 11, if desired. In some embodiments, the distal bearing 14 may exclusively be a radial bearing.

Again, a purge fluid may be supplied through the fluid line 15 of the drive shaft 12 towards a distal bearing 14, pass by the pin 19, which forms a distal radial bearing for the rotor 10, and leave the rotor 10 at its distal end. This prevents blood from entering the rotor 10, reduces friction and cools the distal bearing 14. Alternatively, the distal bearing 14 may not be purged. Accordingly, there may not be a fluid line 15.

Furthermore, in the embodiment shown in Fig. 5, the pin 19 is sitting inside the central duct 15 of the rotor 10, when the housing 11 is expanded. In this case, for example, the drive shaft 12 may terminate at the distal end surface of the rotor 10. Alternatively, the distal end of the drive shaft 12 may be located inside the rotor 10, e.g., at the level of the bottom of the recess as seen in the embodiment of Fig. 4 so as to form the stop for the pin 19.

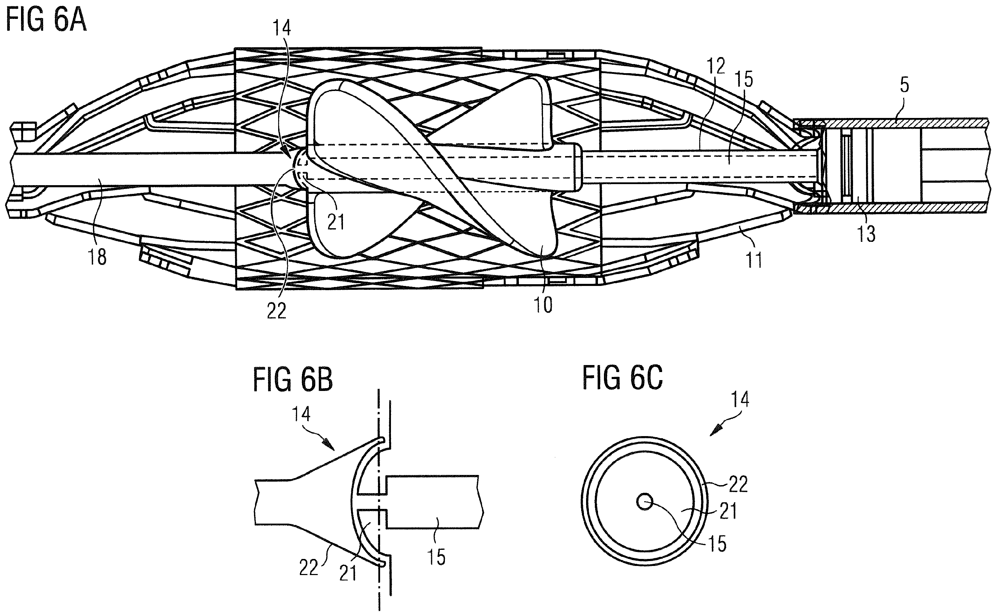

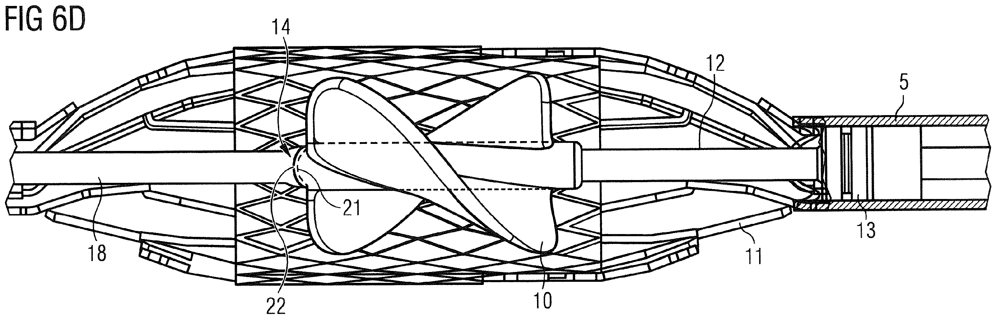

Figs. 6A, 6B, 6C and 6D show a third embodiment of the pump section 4 with the compressible housing 11 and the static support member 18, which is attached to the housing 11. The rotor 10 comprises a nose 21 at its distal end. In Figs. 6A, 6B and 6C, the fluid line 15 inside the distal end of the drive shaft 12 leads to an opening in the nose 21 through which purge fluid may enter the bearing gap of the distal bearing 14 between the nose 21 and a corresponding recess 22 at the proximal end of the static support member 18. In Fig. 6D, however, the distal bearing 14 is unpurged. Thus, the embodiment in Fig. 6D does not possess a fluid line 15 and an opening in the nose 21. The unpurged distal bearing 14 may reduce the amount of purge fluid needed to operate the intravascular blood pump 1. In combination with an unpurged proximal bearing 13, the intravascular blood pump 1 may need no purge fluid at all.

When the housing 11 is compressed, the nose 21 dislodges from the recess 22 and thus the intravascular blood pump 1 becomes more flexible. When the housing 11 is expanded at the target site, the nose 21 automatically moves into the recess 22, wherein the conical or spherical or otherwise converging shape of the nose 21 helps to guide the nose 21 into the recess 22 and centers the rotor 10 with respect to the static support member 18. Fig. 6b shows an enlarged section of the distal bearing 14 with the nose 21 at the rotor 10 and the corresponding recess 22. A vertical dashed and dotted line in Fig. 6b shows the cross-sectional plane of Fig. 6C. The cross-section exhibited in Fig. 6C displays the distal bearing 14 in concentric circles. From periphery to center, the concentric circles show the recess 22, the distal bearing gap between recess 22 and nose 21, the nose 21 and the opening of the fluid line 15 into the distal bearing gap.

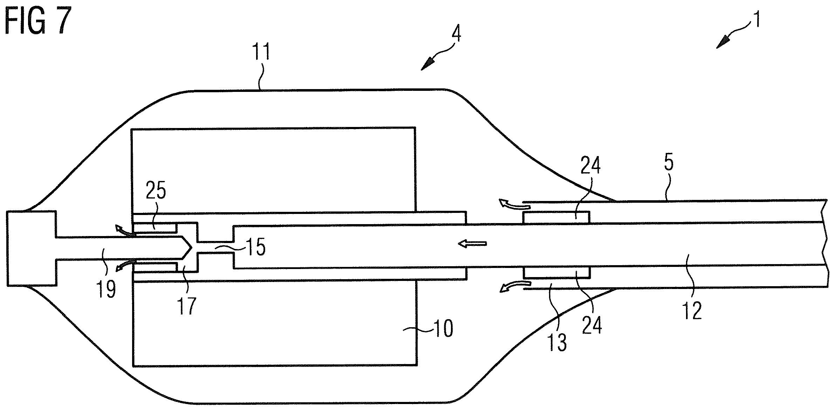

Fig. 7 shows schematically the intravascular blood pump 1 with its catheter 5 and its pump section 4. In this embodiment, the intravascular blood pump 1 comprises a proximal bearing 13 inside the distal end of the catheter 5. Inside the proximal bearing 13, an inner bearing sleeve 24 is glued onto the drive shaft 12 to provide a smooth bearing surface. To fit the bearing sleeve 24, the drive shaft 12 had some of its outer windings removed to reduce its diameter. Purge fluid may now flow through the catheter 5 and exit the proximal bearing 13 through its bearing gap. Some of the purge fluid also flows through the drive shaft 12 into the rotor 10.

The sleeve 24 of the proximal bearing may have an inner diameter preferably ranging from 0.3 mm to 1.5 mm, more preferably from 0.5 mm to 1.2 mm and most preferably from 0.7 mm to 0.9 mm.

The outer diameter of the bearing sleeve 24 of the proximal bearing is preferably between 0.5 mm and 2 mm, more preferably between 0.8 mm and 1.8 mm and most preferably between 0.9 mm and 1.2 mm. The bearing gap of the proximal bearing is preferably between 1 µm and 10 µm, more preferably between 2 µm and 8 µm.

From the drive shaft 12 inside the rotor, the purge fluid flows through the fluid line 15 into the recess 17 of the rotor 10. Arranged inside the recess 17 is the sleeve of the distal bearing 25 of the rotor 10. The inner surface of the sleeve of the distal bearing 25 and the outer surface of the pin 19 form the bearing surfaces of the distal bearing 14. The purge fluid leaves the rotor 10 via the bearing gap between the sleeve of the distal bearing 25 and the pin 19.

The sleeve of the distal bearing 25 has an inner diameter of preferably between 0.3 mm and 1.5 mm, more preferably between 0.5 mm and 1.2 mm and most preferably between 0.7 mm and 0.9 mm. The outer diameter of the sleeve of the distal bearing 25 is preferably between 0.5 mm and 1.7 mm, more preferably between 0.7 mm and 1.4 mm and most preferably between 0.9 mm and 1.1 mm. The bearing gap between the pin 19 and the sleeve of the distal bearing 25 is preferably between 1 µm and 10 µm, more preferably between 2 µm and 8 µm.

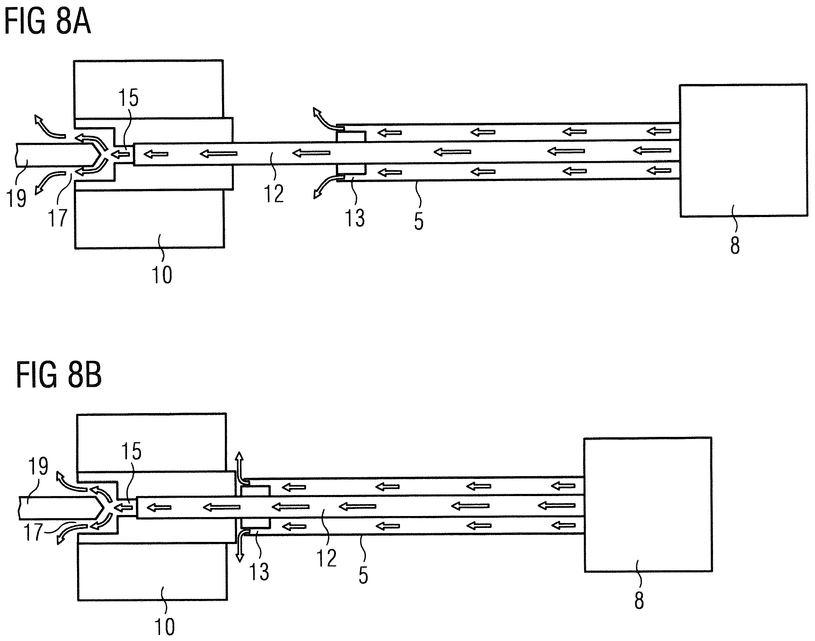

Fig. 8A shows schematically the purge fluid path inside the intravascular blood pump. Inside the housing of the motor 8, the purge fluid is supplied into the catheter 5 and into the drive shaft 12. Herein, the proximal bearing 13 is drawn schematically, its constituent parts, in particular the outer bearing ring 32 and the bearing sleeve 30, are not shown. At the proximal bearing 13, purge fluid leaves the catheter 5 through the bearing gap to reduce friction and cool the proximal bearing 13. A portion of the purge fluid does not leave the catheter 5 through the bearing gap but flows through the drive shaft 12 into the rotor 10. In some embodiments, the drive shaft 12 may comprise a cover such that the purge fluid may flow from the catheter 5 to the rotor 10 without leaking from the drive shaft 12 between the distal end of the catheter 5 and the proximal end of the rotor 10. Inside the rotor 10, the purge fluid continues to flow through the fluid line 15 and then into the recess 17 at the distal end of the rotor 10. In alternative embodiments, the drive shaft 12 may continue up to or into the recess 17 such that the purge fluid flows into the recess 17 directly from the drive shaft 12. From there, the purge fluid flows through the bearing gap of the distal bearing 14 between the pin 19 and the adjacent surface of the rotor 10.

Fig. 8B shows an embodiment of the blood pump similar to Fig. 8A. In Fig. 8B, the proximal bearing 13 is closer to the rotor 10 than in Fig. 8A and is separated from the rotor 10 only by a small gap. Through said gap, purge fluid may escape as shown by arrows.

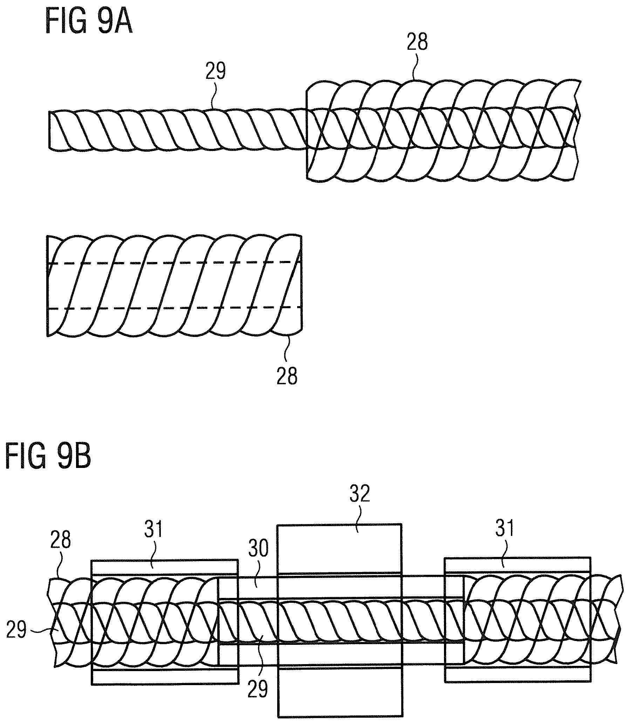

Fig. 9A shows an example of the drive shaft 12 comprising one outer layer 28 and one inner layer 29. In this embodiment, the outer layer 28 and the inner layer 29 consist of helically wound wires, wherein the helix of the inner layer 29 is right-handed and the helix of the outer layer 28 is left-handed. As shown in Fig. 9A, a piece of the outer layer 28 is removed from the inner layer 29 and is shown separately. Removal of the piece of the outer layer 28 may be carried out by pulling the outer layer 28 while turning it slightly. A bearing sleeve 30 may be pushed onto the exposed inner layer 29 until it abuts the outer layer 28, and the piece of outer layer 28 may then be mounted back onto the inner layer 29 adjacent the bearing sleeve 30.

Fig. 9B shows the flexible bearing shaft 12 with the outer layer 28 and the inner layer 29, wherein the outer layer 28 is absent at a central location and the bearing sleeve 30 is situated on the inner layer 29 at the said central location. Furthermore, to both sides of the bearing sleeve 30 and overlapping therewith are two protective rings 31 which are slipped over the ends of the outer layers 28 facing the bearing sleeve 30. A shorter part of the protective rings 31 overlaps the bearing sleeve 30 while a larger part covers the outer layer 28. This way, the risk of breakage of the drive shaft due to a change of stiffness at the transition between the small and large shaft diameters is reduced.

During assembly, the outer layer 28 may be cut and removed from one end of the drive shaft 12. At this point, the drive shaft 12 resembles the representation in Fig. 9A. Thereafter, a first protective ring 31 is placed over the end of the remaining outer layer 28. The bearing sleeve 30 is then placed on top of the inner layer 29, where the outer layer 28 is removed, and overlaps with the protective ring 31. The outer bearing ring 32 is placed on top of the bearing sleeve 30. Then, the previously removed outer layer 28 is again mounted on top of the inner layer 29 with a second protective ring 31 overlapping the end of the outer layer 28 and the bearing sleeve 30. The bearing sleeve 30 and the protective rings 31 may be affixed to the drive shaft 12 using a low-viscosity adhesive. After the adhesive has set, the bearing sleeve 30 may be tested for tightness, i.e., it may be tested whether a purge fluid can pass through the bearing sleeve 30.

The bearing sleeve 30 is rotatably supported in the outer bearing ring 32 which, in turn, is fixed in the catheter or in a proximal end of the housing in which the rotor is housed. The bearing sleeve 30 and the outer bearing ring 32 form a radial bearing while the protective rings 31 form an axial stop and in some embodiments also an axial bearing with the outer bearing ring 32. The bearing sleeve 30 together with the protection rings 31 may be built from a single piece of material. As mentioned, the bearing sleeve 30 and the protective rings 31 are fixedly connected to the drive shaft 12, preferably glued. Glue is also used to fill the windings of inner layer 29 and outer layer 28 to prevent purge fluid from leaking through the drive shaft 12.

In this example, an internal diameter of the bearing sleeve 30 is approximately the same as an outer diameter of the inner layer 29. An outer diameter of the bearing sleeve 30 is approximately the same as an outer diameter of the outer layer 28.

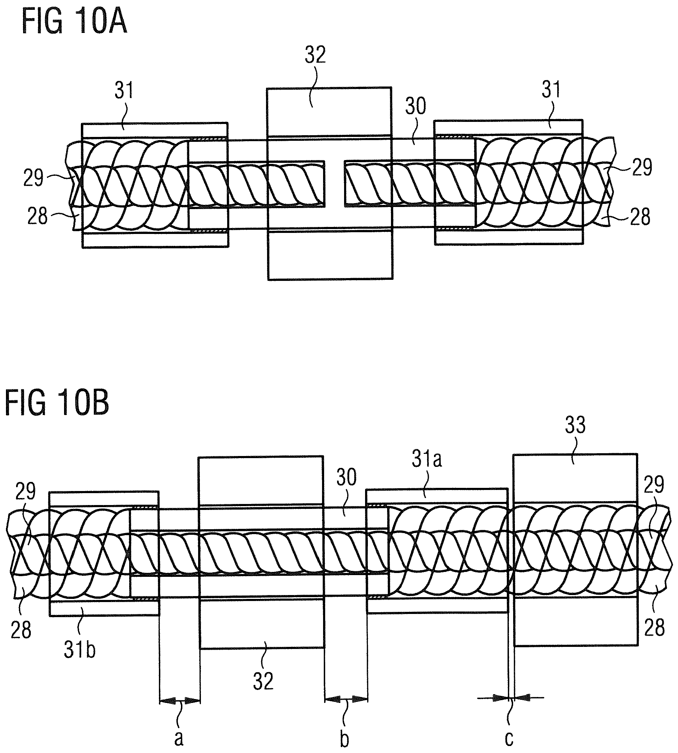

Fig. 10A shows a hydraulically divided bearing sleeve 30 which contains a wall between two blind holes. The inner layer 29 is axially disconnected. Each of the blind holes of the bearing sleeve 30 receives a respective axial end of the axially disconnected inner layer 29. The bearing sleeve 30 does not let any purge fluid pass in an axial direction. Due to this, the inner layer 29 does not need to be filled with glue to prevent any flow of purge fluid through the inner layer 29. Glue may still be used to attach the inner layer 29 to the bearing sleeve 30, but alternative attachment techniques, such as soldering, crimping and welding, are also possible. The outer bearing ring 32 sits on the bearing sleeve 30 and is prevented from being pushed off of the bearing sleeve 30 by the two protective rings 31. Again, the bearing sleeve 30 together with one of the protective rings 31 may be built from a single piece of material.

Fig. 10B shows another embodiment with the outer bearing ring 32 forming a radial bearing with the bearing sleeve 30. Furthermore, a proximal protective ring 31a and a distal protective ring 31b are fixed axially relative to the bearing sleeve 30 in the manner as described before. If the drive shaft 12 moves distally (to the left in Fig. 10B), the proximal protective ring 31a will abut against the proximal surface of the outer bearing ring 32 and any further distal movement is prevented. If the drive shaft 12 moves in a proximal direction, the proximal protective ring 31a will abut against a distal surface of the restriction member 33, stopping any further movement in a proximal direction. If a maximal distance aₘₐₓ between the proximal surface of the distal protective ring 31b and the distal surface of the outer bearing ring 32 is greater than the maximal distance cₘₐₓ between a distal surface of the restriction member 33 and a proximal surface of the proximal protective ring 31a, then the distal protective ring 31b will never touch the outer bearing ring 32. This condition is equivalent to the inequation a > b + c, wherein b + c is constant.

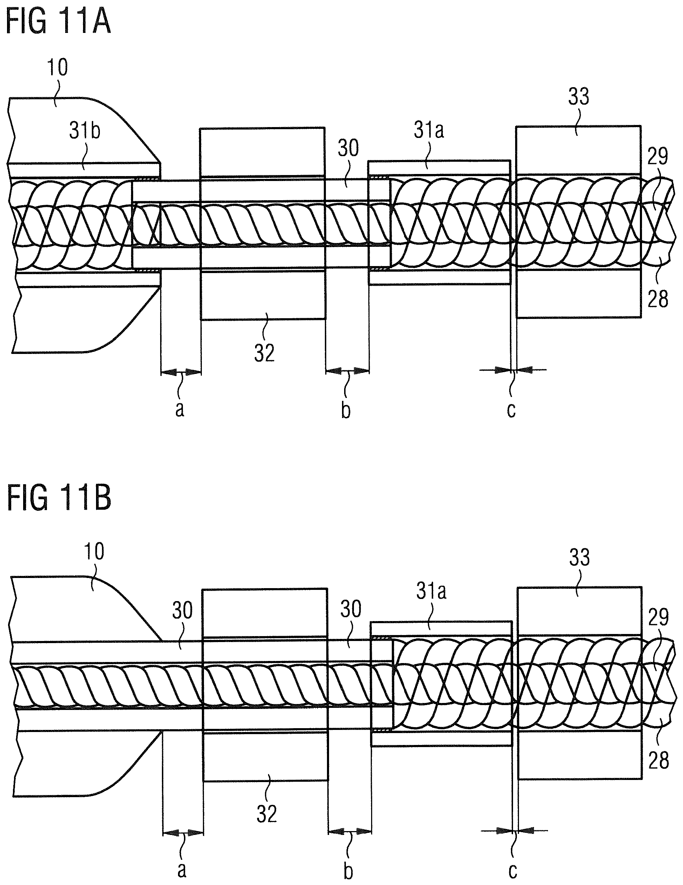

If the rotor 10 is mounted on the distal protective bearing 31b as shown in Fig. 11A, then the distances a, b and c chosen according to the above inequation will prevent the rotor from touching the outer bearing ring 32. Similarly, and as shown in Fig. 11B, if the rotor 10 is mounted on a distal extension of the bearing sleeve 30, the above condition will prevent the rotor 10 on the bearing sleeve 30 from touching the outer bearing ring 32. A touching of the rotor 10 and the outer bearing ring 32 might otherwise cause damage to the rotor 10 or to the proximal bearing 13.

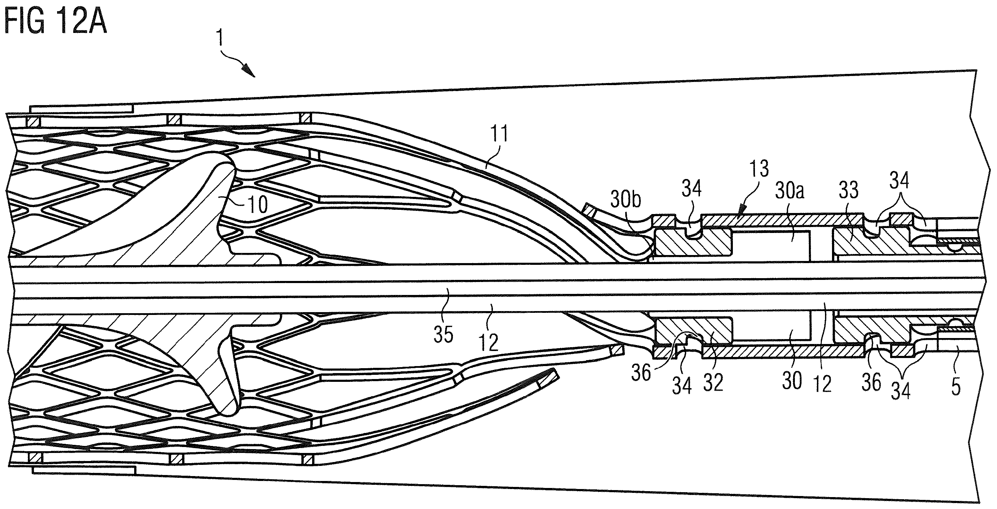

Fig. 12A shows the intravascular blood pump 1 with the housing 11 and the rotor 10 mounted on the drive shaft 12. The proximal bearing 13 comprises the bearing sleeve 30 rotatably supported in the outer bearing ring 32. The drive shaft 12 is glued into bearing sleeve 30. The drive shaft 12 surrounds a reinforcement element 35 implemented as a coaxial rod for stabilizing the distal end of the drive shaft. The rod extends from proximally of the proximal bearing 13 to the distal end of the rotor 10. Alternatively, the drive shaft 12 may be hollow to permit purge fluid to reach the distal bearing. The restriction member 33 is located proximally of the bearing sleeve 30 and prevents the bearing sleeve 30 from dislodging from the outer bearing ring 32. Both the restriction member 33 and the outer bearing ring 32 are press-fitted and/or glued into the distal end of the housing 11. In addition, the restriction member 33 is press-fitted and/or glued into the catheter 5. Thereby, the restriction member 33 connects the housing 11 and the catheter 5. The radial through-holes 34 in the housing 11 serve to introduce glue to fixedly connect the restriction member 33 and the outer bearing ring 32 to the housing 11. The glue may distribute circumferentially along grooves 36 provided in both the restriction member 33 and the outer bearing ring 32. Furthermore, the radial through-holes 34 may be used for position control of the outer bearing ring 32 and the restriction member 33. Both connections are glued in order to keep the connections tight and to prevent leakage of purge fluid.

As can be seen from Fig. 12A, the bearing sleeve 30 comprises a proximal portion 30a located proximally of the outer bearing ring 32 and a distal portion 30b extending from the proximal portion 30a distally into the outer bearing 32. The proximal portion 30a forms an axial bearing with a proximal surface of the outer bearing 32, whereas the distal portion 30b forms a radial bearing with the outer bearing ring 32. The axial bearing and the radial bearing together constitute the proximal bearing 13.

Purge fluid being pressed through the proximal bearing 13 from proximally to distally would first pass the proximal portion 30a of the bearing sleeve 30 along a radial outer surface thereof, then flow radially inwards through the bearing gap between the distal surface of the proximal portion 30a and the proximal surface of the outer bearing ring 32, and finally flow further in a distal direction through the bearing gap formed radially between the distal portion 30b of the bearing sleeve 30 and the radial inner surface of the outer bearing ring 32. The bearing gaps can be designed with little tolerances so that the purge fluid flows through the bearing gaps in a closely controllable manner by applying a suitable pressure on the purge fluid from proximally. A radial notch or radial notches (not shown) may be provided in the proximal surface of the static outer bearing ring 32 to guarantee that purge fluid can flow to the radial bearing gap between the outer bearing ring 32 and the distal portion 30b of the bearing sleeve 30 when, during operation, the rotor 10 pulls the bearing sleeve 32 in a distal direction.

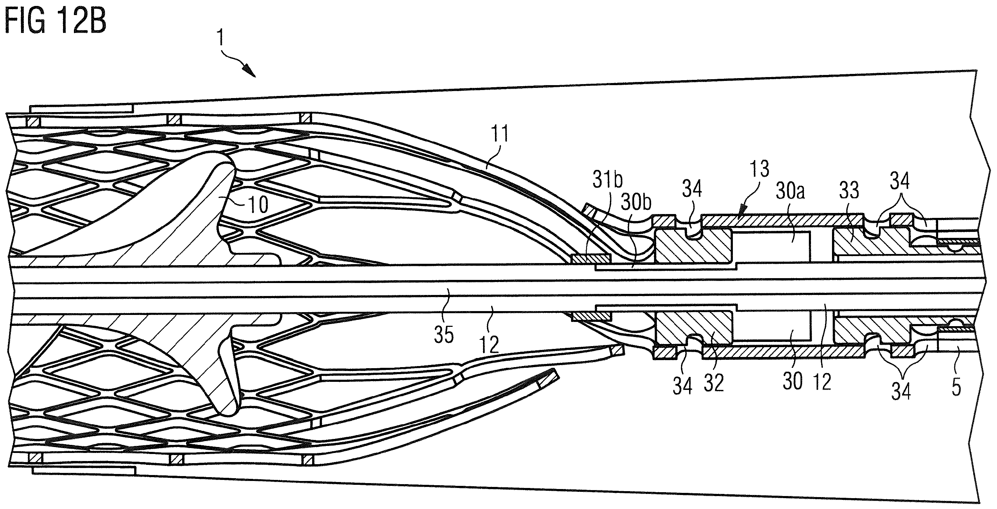

Fig. 12B shows an alternative embodiment to the embodiment of Fig. 12A. Here, the drive shaft 12 has a section of reduced diameter, and the distal portion 30b of the bearing sleeve 30 is arranged at the section of reduced diameter. This way, although not specifically shown in Fig. 12B, the outer diameter of the outer bearing ring 32 can be reduced accordingly, so that, in turn, the outer diameter of the catheter 5 can likewise be reduced. This way, a more flexible and better maneuverable catheter may be achieved.

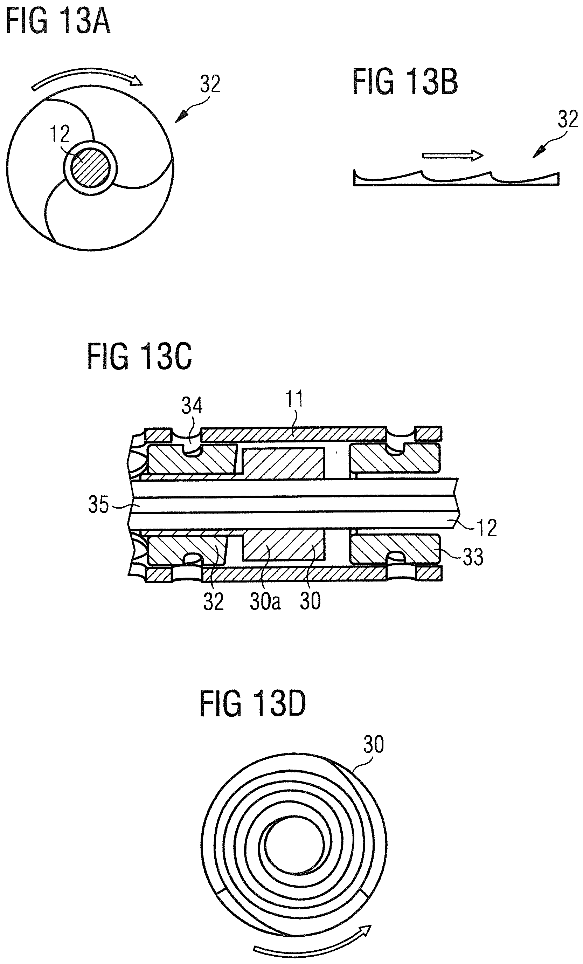

The structure of the bearing sleeve 30 as shown in Fig. 12B is comparable to the bearing structures as described above in relation to Figs. 13 to 15. More specifically, the proximal portion 30a of the bearing sleeve 30 corresponds to the proximal protective ring 31a (see Fig. 10B). Accordingly, the distal bearing ring 31b overlapping both the drive shaft 12 and the distal end of the distal portion 30b of the bearing sleeve 30 is also provided in the embodiment shown in Fig. 12B. It limits the axial movement of the shaft 12 within the outer bearing ring 32 in the same manner as described in relation to Figs. 13 to 15.

Fig. 13A shows a graphical representation of a stationary surface of a hydrodynamic axial bearing. Specifically, Fig. 13A shows the proximal surface of the outer bearing ring 32 with a centrally located drive shaft 12. The curved radial lines in Fig. 13A represent raised portions of the bearing surface, which is shown in more detail in Fig. 13B. The arrows in Fig. 13A and Fig. 13B illustrate the direction of movement of the opposing surface. This then corresponds to the movement direction of the lubricating film within the axial bearing gap. The surface has ramps which form converging gaps together with the opposing stationary surface, which is even. This causes a hydrodynamic pressure to build up in the lubricating film. Thereby, the surfaces forming the axial bearing gap remain at a distance.

Fig. 13C shows the bearing sleeve 30 and the outer bearing ring 32 inside the housing 11. The bearing sleeve 30 has an even distal surface. Here, the opposing proximal surface of the outer bearing ring 32 is slanted to form a convergent gap. During use, this creates the lubricating film required for a hydrodynamic bearing.

Fig. 13D shows spiral grooves in another embodiment of the proximal bearing surface of the outer bearing ring 32. The spiral grooves are preferably configured in the moving surface of the proximal bearing 13, i.e., in the proximal portion 30a of the bearing sleeve 30. In this case, several grooves are positioned in the shape of a spiral in the distal surface of the proximal portion 30a of the bearing sleeve 30. When the bearing sleeve 30 rotates in the direction pointed to by the arrow in Fig. 13D, the lubricating film is transported radially inward along the grooves and forms a pressure between the bearing surfaces, keeping them apart.

Although the invention is only defined by the appended claims, the following aspects of the present disclosure are considered as useful for understanding the invention

According to a first aspect, an intravascular blood pump (1) is disclosed, the pump (1) comprising:
a catheter (5);
a housing (11) in which a rotor (10) is housed, the housing (11) being attached to a distal end of the catheter (5), wherein the housing has an expandable section; and a flexible drive shaft (12) extending through the catheter (5) and connected to the rotor (10), said drive shaft (12) comprising at least one outer layer (28) and at least one inner layer (29),
wherein the drive shaft (12) is rotatably supported in a proximal bearing (13) located proximally of the rotor (10) and wherein the at least one outer layer (28) of the drive shaft (12) is absent or thinned at a location where the drive shaft (12) is supported in the proximal bearing (13).

According to a second aspect that is based on the first aspect, at least one of the at least one inner layer (29) and the at least one outer layer (28) comprises or consists of a wire.

According to a third aspect that is based on the first or second aspect, the drive shaft (12) is at least partly filled with a sealant penetrating into the at least one inner layer (29).

According to a fourth aspect that is based on any one of the first to third aspect, the intravascular blood pump (1) comprises a purge fluid supply line arranged to supply purge fluid such that the purge fluid flows through the proximal bearing (13).

According to a fifth aspect that is based on any one of the first to fourth aspect, at the location where the at least one outer layer (28) of the drive shaft (12) is absent or thinned, a bearing sleeve (30) surrounds the at least one inner layer (29) or the thinned at least one outer layer (28) and said bearing sleeve (30) forms an inner surface of the proximal bearing (13).

According to a sixth aspect that is based on the fifth aspect, the at least one inner layer (29) is axially disconnected inside the bearing sleeve (30).

According to a seventh aspect that is based on the fifth or sixth aspect, the bearing sleeve (30) is fixedly connected to the at least one inner layer (29) or the thinned at least one outer layer (28) of the drive shaft (12).

According to an eight aspect that is based on any one of the fifth to seventh aspect, the bearing sleeve (30) comprises at least one of the following materials: ceramics and metals.

According to a ninth aspect that is based on any one of the fifth to eight aspect, the intravascular blood pump (1) comprises at least one protective ring (31) extending over both the at least one outer layer of the drive shaft (12) and a portion of the bearing sleeve (30).

According to a tenth aspect that is based on the ninth aspect, the protective ring (31) is fixedly connected to the drive shaft (12).

According to an eleventh aspect that is based on the ninth or tenth aspect, the protective ring (31) comprises at least one of the following materials: ceramics and metals.

According to a twelfth aspect that is based on any one of the ninth to eleventh aspect, the protective ring (31) provides an axial bearing surface of the proximal bearing (13).

According to a thirteenth aspect that is based on any one of the ninth to twelfth aspect, two different adhesives are used in the intravascular blood pump (1):
- a first adhesive penetrating the inner layer (29); and
- a second adhesive connecting at least one of the bearing sleeve (30) and the at least one protective ring (31) to the drive shaft (12).

According to a fourteenth aspect that is based on any one of the ninth to thirteenth aspect, the bearing sleeve (30) extends into the rotor (10).

According to a fifteenth aspect that is based on any one of the first to fourteenth aspect, a radial bearing gap between the outer bearing ring (32) and the bearing sleeve (30) is between 1 µm and 10 µm, preferably between 2 µm and 8 µm wide.

## Claims

1. An intravascular blood pump (1), comprising:
a catheter (5),
a housing (11) in which a rotor (10) is housed, the housing (11) being attached to a distal end of the catheter (5), wherein the housing has an expandable section, and
a flexible drive shaft (12) extending through the catheter (5), connected to the rotor (10) and rotatably supported in a proximal bearing (13) located proximally of the rotor (10),
**characterized in that** the drive shaft (12) comprises at least one outer layer (28) and at least one inner layer (29), and the at least one outer layer (28) of the drive shaft (12) is absent or thinned at a location where the drive shaft (12) is supported in the proximal bearing (13).

2. A method of manufacturing an intravascular blood pump (1) which comprises:
- a catheter (5);
- a housing (11) in which a rotor (10) is housed, the housing (11) being attached to a distal end of the catheter (5); and
- a flexible drive shaft (12) extending through the catheter (5) and connected to the rotor (10), said drive shaft (12) comprising at least one outer layer (28) and at least one inner layer (29), wherein the drive shaft (12) is rotatably supported in a proximal bearing (13) located proximally of the rotor (10),
wherein the method comprises the step of thinning or completely removing the at least one outer layer (28) of the drive shaft (12) at a location where the drive shaft (12) is supported in the proximal bearing (13).

3. The method according to claim 2, comprising the step of at least partly filling the drive shaft (12) with a sealant so that the sealant penetrates into the at least one inner layer (29).

4. The method according to claim 3, wherein the step of at least partly filling the drive shaft (12) with a sealant is carried out such that the sealant penetrates into all layers, i.e. into the at least one inner layer (29) and the at least one outer layer (28).

5. The method according to claim 3 or 4, wherein the sealant is a substance which is configured to penetrate the layers (28, 29) as a fluid and then hardens to prevent penetration of purge fluid through the respective layers (28, 29).

6. The method according to any one of claims 2 to 5, wherein the method comprises the step of providing, at the location where the at least one outer layer (28) of the drive shaft (12) is removed or thinned, a bearing sleeve (30) so that the bearing sleeve (30) surrounds the at least one inner layer (29) or the thinned at least one outer layer (28) and forms an inner surface of the proximal bearing (13).

7. The method according to claim 6, wherein the at least one inner layer (29) is axially disconnected inside the bearing sleeve (30), wherein the method comprises the step of fixing the bearing sleeve (30) on top of the at least one inner layer (29).

8. The method according to claim 6, wherein the at least one inner layer (29) is not axially disconnected inside the bearing sleeve (30), wherein the method comprises the step of feeding the at least one inner layer (29) through the bearing sleeve before mounting a next following section of the at least one outer layer (28) on the at least one inner layer (29).

9. The method according to claim 6, wherein the at least one inner layer (29) is axially disconnected inside the bearing sleeve (30) and the bearing sleeve (30) is a cylinder with a wall separating two blind holes so that an internal space of the bearing sleeve (30) is hydraulically separated so that liquid cannot flow internally from one side of the bearing sleeve (30) to another, wherein the method comprises the step of inserting each of two ends of the axially disconnected at least one inner layer (29) into a respective one of the blind holes.

10. The method according to any one of claims 6 to 9, wherein the method comprises the step of fixedly connecting the bearing sleeve (30) to the at least one inner layer (29) or the thinned at least one outer layer (28) of the drive shaft (12) by crimping, soldering, welding, gluing or shrinking the bearing sleeve (30) to the at least one inner layer (29) or to the thinned at least one outer layer (28).

11. The method according to any one of claims 6 to 10, wherein the method comprises the step of providing one or more protective rings (31) so as to extend over both the at least one outer layer (28) of the drive shaft (12) and a respective portion of the bearing sleeve (30).

12. The method according to claim 11, comprising the steps of providing a distal protective ring (31b) so as to extend over the drive shaft (12) and a distal portion of the bearing sleeve (30) and a proximal protective ring (31a) so as to extend over the drive shaft (12) and a proximal portion of the bearing sleeve.

13. The method according to claim 12, wherein the method comprises the step of placing the proximal protective ring (31a) axially between an outer bearing ring (32) and a restriction member (33) so that the restriction member (33) acts as a limit restricting axial movement of the drive shaft (12) relative to the outer bearing ring (32).

14. The method according to claim 13, comprising the step of mounting the rotor (10) or a shaft of the rotor (10) distally of the outer bearing ring (32) so as to form the distal protective ring (31b), in which case the restriction member (33) prevents touching of the rotor (10) or rotor shaft and the outer bearing ring (32).

15. The method according to claim 13 or 14, comprising the step of fixing the outer bearing ring (32) to at least one of the catheter (5) and a proximal section of the housing (11).

## Patentansprüche

1. Intravaskuläre Blutpumpe (1), aufweisend:
einen Katheter (5),
ein Gehäuse (11), in dem ein Rotor (10) untergebracht ist, wobei das Gehäuse (11) an einem distalen Ende des Katheters (5) angebracht ist, wobei das Gehäuse einen expandierbaren Abschnitt aufweist, und
eine flexible Antriebswelle (12), die sich durch den Katheter (5) erstreckt, mit dem Rotor (10) verbunden ist und drehbar in einem proximalen Lager (13) gelagert ist, das proximal des Rotors (10) angeordnet ist,
**dadurch gekennzeichnet, dass** die Antriebswelle (12) mindestens eine äußere Schicht (28) und mindestens eine innere Schicht (29) aufweist, und die mindestens eine äußere Schicht (28) der Antriebswelle (12) an einer Stelle fehlt oder verdünnt ist, an der die Antriebswelle (12) in dem proximalen Lager (13) gelagert ist.

2. Verfahren zur Herstellung einer intravaskulären Blutpumpe (1), die das Folgende aufweist:
- einen Katheter (5);
- ein Gehäuse (11), in dem ein Rotor (10) untergebracht ist, wobei das Gehäuse (11) an einem distalen Ende des Katheters (5) angebracht ist; und
- eine flexible Antriebswelle (12), die sich durch den Katheter (5) erstreckt und mit dem Rotor (10) verbunden ist, wobei die Antriebswelle (12) mindestens eine äußere Schicht (28) und mindestens eine innere Schicht (29) aufweist, wobei die Antriebswelle (12) drehbar in einem proximalen Lager (13) gelagert ist, das proximal des Rotors (10) angeordnet ist,
wobei das Verfahren den Schritt aufweist, die mindestens eine äußere Schicht (28) der Antriebswelle (12) an einer Stelle, an der die Antriebswelle (12) in dem proximalen Lager (13) gelagert ist, zu verdünnen oder vollständig zu entfernen.

3. Verfahren nach Anspruch 2, wobei das Verfahren den Schritt aufweist, die Antriebswelle (12) zumindest teilweise mit einem Dichtmittel zu füllen, so dass das Dichtmittel in die mindestens eine innere Schicht (29) eindringt.

4. Verfahren nach Anspruch 3, wobei der Schritt des zumindest teilweisen Füllens der Antriebswelle (12) mit einem Dichtmittel so durchgeführt wird, dass das Dichtmittel in alle Schichten eindringt, d. h. in die mindestens eine innere Schicht (29) und in die mindestens eine äußere Schicht (28).

5. Verfahren nach Anspruch 3 oder 4, wobei das Dichtmittel eine Substanz ist, die so ausgestaltet ist, dass sie als Fluid in die Schichten (28, 29) eindringt und anschließend aushärtet, um ein Eindringen von Spülflüssigkeit durch die jeweiligen Schichten (28, 29) zu verhindern.

6. Verfahren nach einem der Ansprüche 2 bis 5, wobei das Verfahren den Schritt aufweist, an der Stelle, an der die mindestens eine äußere Schicht (28) der Antriebswelle (12) entfernt oder verdünnt ist, eine Lagerhülse (30) bereitzustellen, so dass die Lagerhülse (30) die mindestens eine innere Schicht (29) oder die gedünnte mindestens eine äußere Schicht (28) umgibt und eine Innenfläche des proximalen Lagers (13) bildet.

7. Verfahren nach Anspruch 6, wobei die mindestens eine innere Schicht (29) innerhalb der Lagerhülse (30) axial abgekoppelt ist, wobei das Verfahren den Schritt aufweist, die Lagerhülse (30) auf der mindestens einen inneren Schicht (29) zu befestigen.

8. Verfahren nach Anspruch 6, wobei die mindestens eine innere Schicht (29) innerhalb der Lagerhülse (30) nicht axial abgekoppelt ist, wobei das Verfahren den Schritt aufweist, die mindestens eine innere Schicht (29) durch die Lagerhülse zu führen, bevor ein nächster folgender Abschnitt der mindestens einen äußeren Schicht (28) auf der mindestens einen inneren Schicht (29) montiert wird.

9. Verfahren nach Anspruch 6, wobei die mindestens eine innere Schicht (29) innerhalb der Lagerhülse (30) axial abgekoppelt ist und die Lagerhülse (30) ein Zylinder mit einer Wand ist, die zwei Sacklöcher voneinander trennt, sodass ein Innenraum der Lagerhülse (30) hydraulisch getrennt ist, sodass Flüssigkeit nicht intern von einer Seite der Lagerhülse (30) zur anderen fließen kann, wobei das Verfahren den Schritt aufweist, jeweils eines der beiden Enden der axial abgekoppelten mindestens einen inneren Schicht (29) in eines der Sacklöcher einzusetzen.

10. Verfahren nach einem der Ansprüche 6 bis 9, wobei das Verfahren den Schritt aufweist, die Lagerhülse (30) fest mit der mindestens einen inneren Schicht (29) oder der verdünnten mindestens einen äußeren Schicht (28) der Antriebswelle (12) zu verbinden, indem die Lagerhülse (30) an die mindestens eine innere Schicht (29) oder die gedünnte mindestens eine äußere Schicht (28) gecrimpt, gelötet, geschweißt, geklebt oder geschrumpft wird.

11. Verfahren nach einem der Ansprüche 6 bis 10, wobei das Verfahren den Schritt aufweist, einen oder mehrere Schutzringe (31) bereitzustellen, sodass sich diese über beide der mindestens einen äußeren Schicht (28) der Antriebswelle (12) und einem entsprechenden Abschnitt der Lagerhülse (30) erstrecken.

12. Verfahren nach Anspruch 11, wobei das Verfahren die Schritte aufweist, einen distalen Schutzring (31b) bereitzustellen, sodass sich dieser über die Antriebswelle (12) und einen distalen Abschnitt der Lagerhülse (30) erstreckt, und einen proximalen Schutzring (31a) bereitzustellen, sodass sich dieser über die Antriebswelle (12) und einen proximalen Abschnitt der Lagerhülse erstreckt.

13. Verfahren nach Anspruch 12, wobei das Verfahren den Schritt aufweist, den proximalen Schutzring (31a) axial zwischen einem äußeren Lagerring (32) und einem Begrenzungselement (33) zu platzieren, sodass das Begrenzungselement (33) als Begrenzung fungiert, der eine axiale Bewegung der Antriebswelle (12) relativ zu dem äußeren Lagerring (32) begrenzt.

14. Verfahren nach Anspruch 13, wobei das Verfahren den Schritt aufweist, den Rotor (10) oder eine Welle des Rotors (10) distal des äußeren Lagerrings (32) zu montieren, um den distalen Schutzring (31b) zu bilden, wobei das Begrenzungselement (33) in diesem Fall ein Berühren des Rotors (10) bzw. der Rotorwelle mit dem äußeren Lagerring (32) verhindert.

15. Verfahren nach Anspruch 13 oder 14, wobei das Verfahren den Schritt aufweist, den äußeren Lagerring (32) an mindestens einem des Katheters (5) und eines proximalen Abschnitts des Gehäuses (11) zu fixieren.

## Revendications

1. Pompe sanguine intravasculaire (1), comprenant :
un cathéter (5) ;
un boîtier (11), où un rotor (10) est logé, le boîtier (11) étant fixé sur une extrémité distale du cathéter (5), dans laquelle le boîtier présente une section agrandissable, et
un arbre d'entraînement flexible (12) s'étendant à travers le cathéter (5), relié au rotor (10) et supporté de manière à permettre la rotation dans un palier proximal (13) situé de manière proximale par rapport au rotor (10) ;
**caractérisée en ce que** l'arbre d'entraînement (12) comprend au moins une couche extérieure (28) et au moins une couche intérieure (29), et la au moins une couche extérieure (28) de l'arbre d'entraînement (12) est absente ou amincie sur un endroit où l'arbre d'entraînement (12) est supporté dans le palier proximal (13).

2. Procédé de fabrication d'une pompe sanguine intravasculaire (1), laquelle comprend :
un cathéter (5) ;
un boîtier (11), où un rotor (10) est logé, le boîtier (11) étant fixé sur une extrémité distale du cathéter (5),
un arbre d'entraînement flexible (12) s'étendant à travers le cathéter (5), relié au rotor (10), ledit arbre d'entraînement (12) comprenant au moins une couche extérieure (28) et au moins une couche intérieure (29), dans lequel l'arbre d'entraînement (12) est supporté de manière à permettre la rotation dans un palier proximal (13) situé de manière proximale par rapport au rotor (10) ;
dans lequel le procédé comprend l'étape pour amincir ou entièrement retirer la au moins une couche extérieure (28) de l'arbre d'entraînement (12) sur un endroit où l'arbre d'entraînement (12) est supporté dans le palier proximal (13).

3. Procédé selon la revendication 2, comprenant l'étape pour remplir au moins partiellement l'arbre d'entraînement (12) avec un produit d'étanchéité de sorte que le produit d'étanchéité pénètre jusque dans la au moins une couche intérieure (29).

4. Procédé selon la revendication 3, dans lequel l'étape pour remplir au moins partiellement l'arbre d'entraînement (12) avec un produit d'étanchéité est réalisée de telle sorte que le produit d'étanchéité pénètre dans toutes les couches, à savoir jusque dans la au moins une couche intérieure (29) et la au moins une couche extérieure (28).

5. Procédé selon la revendication 3 ou 4, dans lequel le produit d'étanchéité est une substance, laquelle est configurée pour pénétrer dans les couches (28, 29) comme fluide, puis durcir afin d'empêcher la pénétration du fluide de purge à travers les couches respectives (28, 29).

6. Procédé selon l'une quelconque des revendications 2 à 5, dans lequel le procédé comprend l'étape pour fournir, à l'endroit où la au moins une couche extérieure (28) de l'arbre d'entraînement (12) est retirée ou amincie, un palier à douille (30), de sorte que le palier à douille (30) entoure la au moins une couche intérieure (29) ou la au moins une couche extérieure amincie (28), et forme une surface interne du palier proximal (13).

7. Procédé selon la revendication 6, dans lequel la au moins une couche intérieure (29) est désolidarisée axialement dans le palier à douille (30), et dans lequel le procédé comprend l'étape pour fixer le palier à douille (30) sur le dessus de la au moins une couche intérieure (29).

8. Procédé selon la revendication 6, dans lequel la au moins une couche intérieure (29) n'est pas désolidarisée axialement dans le palier à douille (30), et dans lequel le procédé comprend l'étape pour faire passer la au moins une couche intérieure (29) à travers le palier à douille avant de monter une section suivante adjacente de la au moins une couche extérieure (28) sur la au moins une couche intérieure (29).

9. Procédé selon la revendication 6, dans lequel la au moins une couche intérieure (29) est désolidarisée axialement dans le palier à douille (30), et le palier à douille (30) est un cylindre présentant une paroi séparant deux trous borgnes de sorte qu'un espace interne du palier à douille (30) est séparé par voie hydraulique de manière à ce que le liquide ne puisse pas circuler à l'intérieur d'un côté du palier à douille (30) à l'autre, et dans lequel le procédé comprend l'étape pour introduire chacune des deux extrémités de la au moins une couche intérieure désolidarisée axialement (29) jusque dans un trou respectif parmi les trous borgnes.

10. Procédé selon l'une quelconque des revendications 6 à 9, dans lequel le procédé comprend l'étape pour relier de manière fixe le palier à douille (30) à la au moins une couche intérieure (29) ou la au moins une couche extérieure amincie (28) de l'arbre d'entraînement (12) par sertissage, brasage, soudage, collage ou rétrécissement du palier à douille (30) sur la au moins une couche intérieure (29) ou la au moins une couche extérieure amincie (28).

11. Procédé selon l'une quelconque des revendications 6 à 10, dans lequel le procédé comprend l'étape pour fournir un ou plusieurs anneaux protecteurs (31) de manière à ce qu'ils s'étendent à la fois sur la au moins une couche extérieure (28) de l'arbre d'entraînement (12) et sur une portion respective du palier à douille (30).

12. Procédé selon la revendication 11, comprenant l'étape pour fournir un anneau protecteur distal (31b) de manière à ce que celui-ci s'étende sur l'arbre d'entraînement (12) et une portion distale du palier à douille (30), et un anneau protecteur proximal (31a) de manière à ce que celui-ci s'étende sur l'arbre d'entraînement (12) et une portion proximale du palier à douille.

13. Procédé selon la revendication 12, dans lequel le procédé comprend l'étape pour placer l'anneau protecteur proximal (31a) axialement entre un anneau de palier extérieur (32) et un élément restrictif (33) de sorte que l'élément restrictif (33) agit comme une limite restreignant le déplacement axial de l'arbre d'entraînement (12) par rapport à l'anneau de palier extérieur (32).

14. Procédé selon la revendication 13, comprenant l'étape pour monter le rotor (10) ou un arbre du rotor (10) de manière distale par rapport à l'anneau de palier extérieur (32) de manière à former l'anneau protecteur distal (31b), auquel cas l'élément restrictif (33) empêche le contact du rotor (10) ou de l'arbre de rotor et de l'anneau de palier extérieur (32).

15. Procédé selon la revendication 13 ou 14, comprenant l'étape pour fixer l'anneau de palier extérieur (32) sur au moins un élément parmi le cathéter (5) et une section proximale du boîtier (11).
